# EUROPÄISCHE PATENTSCHRIFT

(11) **EP 1 028 963 B1**
(45) Veröffentlichungstag und Bekanntmachung des Hinweises auf die Patenterteilung: **15.06.2005**
(21) Anmeldenummer: 98956894.4
(22) Anmeldetag: 29.10.1998
(51) Int. Cl.: C07D 491/107, A01N 43/90, C07D 309/14, C07C 57/58, C07C 69/63

(54) **NEUE SUBSTITUIERTE PHENYLKETOENOLE**
NOVEL SUBSTITUTED PHENYL KETO ENOLS
NOUVEAUX PHENYLCETOENOLS SUBSTITUES

(30) Priorität: 11.11.1997 DE 19749720
(43) Veröffentlichungstag der Anmeldung: 23.08.2000
(60) Teilanmeldung: 04028198.2
(73) Patentinhaber: Bayer CropScience AG, 40789 Monheim (DE)
(72) Erfinder: HAGEMANN, Hermann, D-51375 Leverkusen (DE); FISCHER, Reiner, D-40789 Monheim (DE); ERDELEN, Christoph, D-42799 Leichlingen (DE); WACHENDORFF-NEUMANN, Ulrike, D-56566 Neuwied (DE); SCHNEIDER, Udo, D-51373 Leverkusen (DE); ANDERSCH, Wolfram, D-51469 Bergisch Gladbach (DE)
(86) Internationale Anmeldenummer: PCT/EP1998/006866
(87) Internationale Veröffentlichungsnummer: WO 1999/024437

(56) Entgegenhaltungen:
- DE-A- 4 415 334

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Bekannt sind 1H-Arylpyrrolidin-dion-Derivate (EP-A-456 063, EP-A-521 334, EP-A-596298, EP-A-613 884, EP-A-613 885, DE-44 40 594, DE-196 49 665, WO 94/01 997, WO 95/01 358, WO 95/20 572, EP-A-668 267, WO 95/26 954, WO 96/25395, WO 96/35 664, WO 97/01 535 und WO 97/02 243) und ihre Verwendung als Schädlingsbekämpfungsmittel und zum Teil als Herbizide.

Die herbizide, akarizide und insektizide Wirksamkeit und/oder Wirkungsbreite und/oder die Pflanzenverträglichkeit dieser Verbindungen, insbesondere gegenüber Kulturpflanzen, ist jedoch nicht immer ausreichend.

Es wurden nun neue Verbindungen der Formel (I) 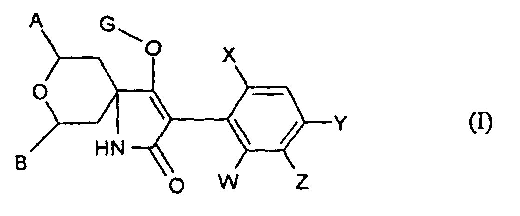 gefunden,
in welcher
- W: für Wasserstoff, Nitro, Cyano, Halogen, C₁-C₆-Alkyl, C₂-C₆-Alkenyl, C₂-C₆-Alkinyl, C₁-C₆-Alkoxy, C₁-C₄-Halogenalkyl oder C₁-C₄-Halogenalkoxy steht,
- X: für Halogen, C₁-C₆-Alkyl, C₂-C₆-Alkenyl, C₂-C₆-Alkinyl, C₁-C₆-Alkoxy, C₃-C₆-Alkenyloxy, C₁-C₄-Halogenalkyl, C₁-C₄-Halogen-alkoxy, C₂-C₆-Halogenalkenyloxy, Cyano, Nitro oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
- Y: für Wasserstoff, Halogen, C₁-C₆-Alkyl, C₁-C₆-Alkoxy, C₁-C₄-Halogenalkyl, C₁-C₄-Halogenalkoxy, Cyano oder Nitro steht,
- Z: für Wasserstoff, Halogen, C₁-C₆-Alkyl, C₁-C₆-Alkoxy, C₁-C₄-Halogenalkyl, C₁-C₄-Halogenalkoxy, Hydroxy, Cyano, Nitro oder für jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht,
- A: für C₁-C₆-Alkyl steht,
- B: für Wasserstoff oder C₁-C₆-Alkyl steht,
- G: für Wasserstoff (a) oder für einen der Reste 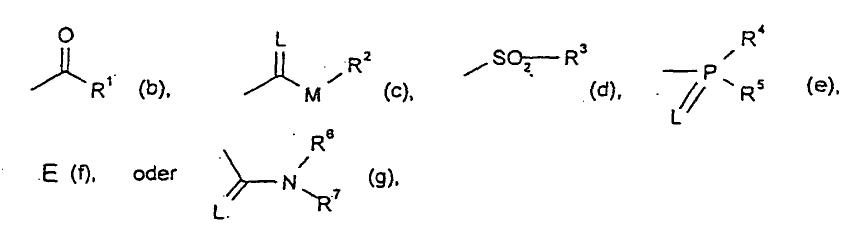
steht, in welchen
- E: für ein Metallionäquivalent oder ein Ammoniumion steht,
- L: für Sauerstoff oder Schwefel steht und
- M: für Sauerstoff oder Schwefel steht.

- R¹: für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff ,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
- R²: für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
- R³: für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di- (C₁-C₈-alkyl)amino, C₁-C₈-Alkylthio oder C₃-C₈-Alkenylthio oder für jeweils gegebenenfalls durch Halogen, Nitro; Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
- R⁶ und R⁷: unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl, C₁-C₈-Alkoxy-C₂-C₈-alkyl oder für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g) 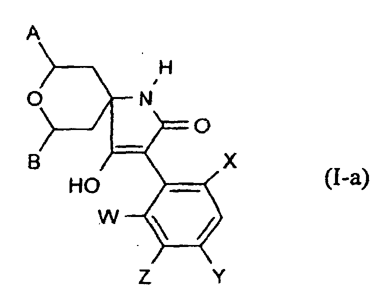 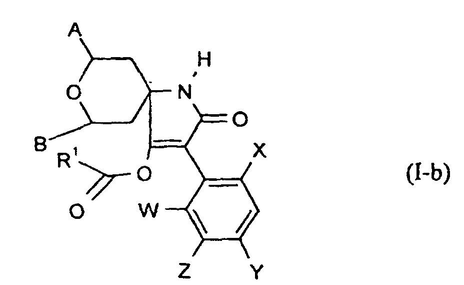 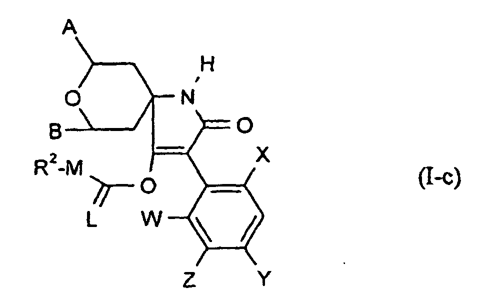 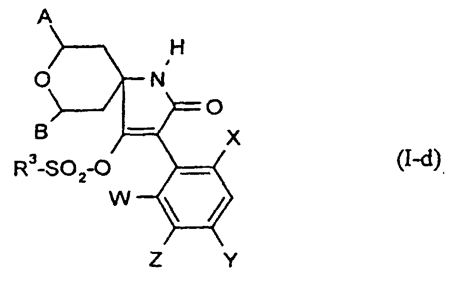 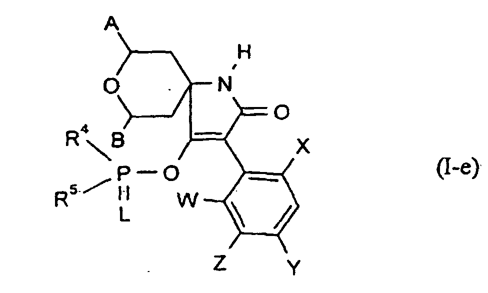 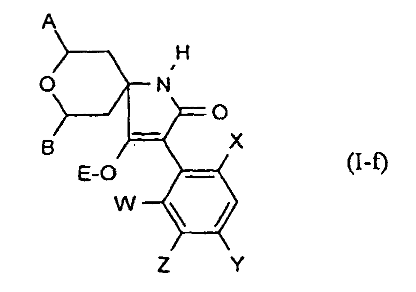 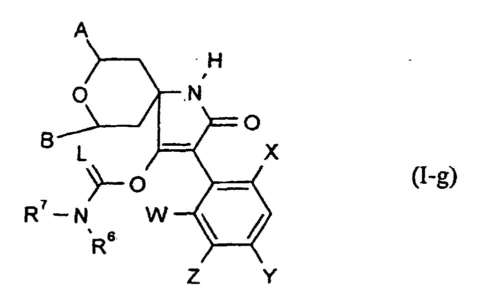 worin
A, B, E, L, M, W, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach den im folgenden beschriebenen Verfahren erhält:
(A) Man erhält Verbindungen der Formel (I-a) 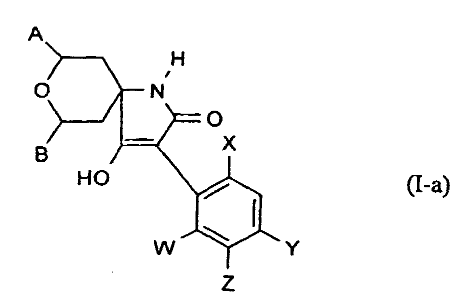 in welcher
   A, B, W, X, Y und Z die oben angegebenen Bedeutungen haben,
   wenn man
   Verbindungen der Formel (II) 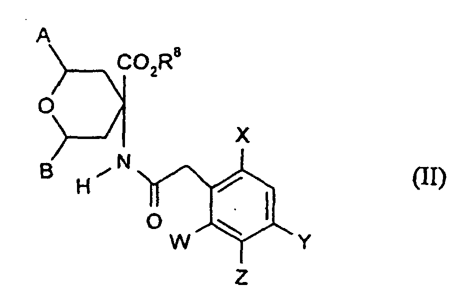 in welcher
   A, B, W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Außerdem wurde gefunden,
(B) daß man die Verbindungen der oben gezeigten Formel (I-b), in welcher R¹, A, B, W, X, Y und Z die oben angebenen Bedeutungen haben, erhält, wenn man Verbindungen der oben gezeigten Formel (I-a), in welcher A, B, W, X, Y und Z die oben angegebenen Bedeutungen haben,
   α) mit Säurehalogeniden der Formel (III) 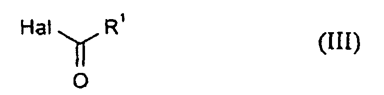 in welcher
      - R¹: die oben angegebene Bedeutung hat und
      - Hal: für Halogen (insbesondere Chlor oder Brom) steht
      oder
   β) mit Carbonsäureanhydriden der Formel (IV)

   R¹-CO-O-CO-R¹ (IV)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(C) daß man die Verbindungen der oben gezeigten Formel (I-c), in welcher R², A, B, W, M, X, Y und Z die oben angegebenen Bedeutungen haben und L für Sauerstoff steht, erhält, wenn man Verbindungen der oben gezeigten Formel (I-a), in welcher A, B, W, X, Y und Z die oben angegebenen Bedeutungen haben,
   mit Chlorameisensäureestern oder Chlorameisensäurethioestern der Formel (V)

   R²-M-CO-Cl (V)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(D) daß man Verbindungen der oben gezeigten Formel (I-c), in welcher R², A, B, W, M, X, Y und Z die oben angegebenen Bedeutungen haben und L für Schwefel steht, erhält, wenn man Verbindungen der oben gezeigten Formel (I-a), in welcher A, B, W, X, Y und Z die oben angegebenen Bedeutungen haben,
   mit Chlormonothioameisensäureestern oder Chlordithioameisensäureestern der Formel (VI) 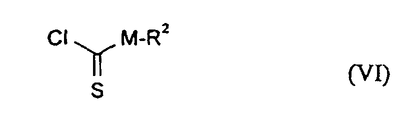 in welcher
   M und R² die oben angegebenen Bedeutungen haben,
   gegebenenfalls in Gegenwart eines Verdünnungsmittels und gegebenenfalls in Gegenwart eines Säurebindemittels umsetzt;
(E) daß man Verbindungen der oben gezeigten Formel (I-d), in welcher R³, A, B, W, X, Y und Z die oben angegebenen Bedeutungen haben, erhält, wenn man Verbindungen der oben gezeigten Formel (I-a), in welcher A, B, W, X, Y und Z die oben angegebenen Bedeutungen haben,
   mit Sulfonsäurechloriden der Formel (VII)

   R³-SO₂-Cl (VII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(F) daß man Verbindungen der oben gezeigten Formel (I-e), in welcher L, R⁴, R⁵, A, B, W, X, Y und Z die oben angegebenen Bedeutungen haben, erhält, wenn man Verbindungen der oben gezeigten Formel (I-a), in welcher A, B, W; X, Y und Z die oben angegebenen Bedeutungen haben,
   mit Phosphorverbindungen der Formel (VIII) 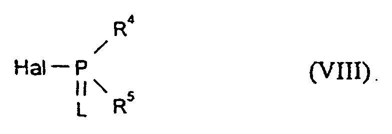 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt;
(G) daß man Verbindungen der oben gezeigten Formeln (I-f), in welcher E, A, B, W, X, Y und Z die oben angegebenen Bedeutungen haben, erhält, wenn man Verbindungen der Formel (I-a), in welcher A, B, W, X, Y und Z die oben angegebenen Bedeutungen haben,
   mit Metallverbindungen oder Aminen der Formeln (IX) oder (X)

   Me(OR⁹)ₜ (IX)

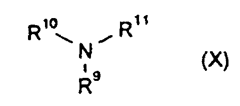

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R⁹, R¹⁰, R¹¹: unabhängig voneinander für Wasserstoff oder Alkyl (bevorzugt C₁-C₈-Alkyl) stehen,
   gegebenenfalls in Gegenwart eines Verdünnungsmittels umsetzt;
(H) daß man Verbindungen der oben gezeigten Formel (I-g), in welcher L, R⁶, R⁷, A, B, W, X, Y und Z die oben angegebenen Bedeutungen haben, erhält, wenn man Verbindungen der oben gezeigten Formel (I-a), in welcher A, B, W, X, Y und Z die oben angegebenen Bedeutungen haben,
   α) mit Isocyanaten oder Isothiocyanaten der Formel (XI)

      R⁶-N=C=L (XI)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XII) 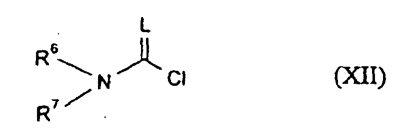
   in welcher
   L, R⁶ und R⁷ die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide und Akarizide und als Herbizide aufweisen und darüber hinaus häufig sehr gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- W: steht besonders bevorzugt für Wasserstoff, Nitro, Cyano, Fluor, Chlor, Brom, C₁-C₄-Alkyl, C₁-C₄-Alkoxy, C₁-C₂-Halogenalkyl oder C₁-C₂-Halogenalkoxy.
- X: steht besonders bevorzugt für Fluor, Chlor, Brom, C₁-C₄-Alkyl, C₁-C₄-Alkoxy, C₃-C₄-Alkenyloxy, C₁-C₂-Halogenalkyl, C₁-C₂-Halogenalkoxy, C₂-C₄-Halogenalkenyloxy, Cyano, Nitro oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl oder Benzyloxy.
- Y: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro.
- Z: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy.
- A: steht besonders bevorzugt für C₁-C₄-Alkyl
- B: steht besonders bevorzugt für Wasserstoff, Methyl oder Ethyl.
- G: steht besonders bevorzugt für Wasserstoff (a) oder für einen der Reste 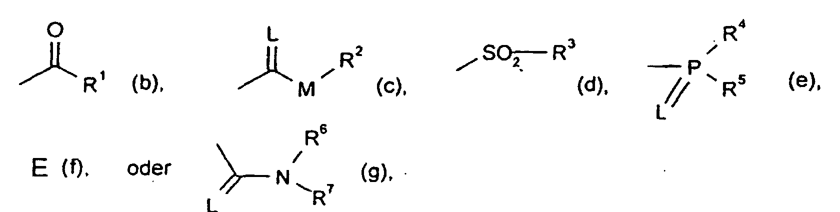 (insbesondere für einen der Reste (a), (b) oder (c)),
in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C ₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy-substituiertes Phenyl oder Benzyl.
- R³: steht besonders bevorzugt für gegebenenfalls durch Fluor oder Chlor substituiertes C₁-C₆-Alkyl oder für jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl oder für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als (besonders) bevorzugt bezeichneten Restedefinitionen steht Halogen in Verbindung mit anderen Resten (beispielsweise in Halogenalkyl, Halogenalkoxy oder Halogenalkenyloxy) insbesondere für Fluor, Chlor und Brom, hervorgehoben für Fluor und Chlor.
- W: steht ganz besonders bevorzugt für Wasserstoff, Nitro, Cyano, Fluor, Chlor, Brom, Methyl, Ethyl, n-Propyl, iso-Propyl, n-Butyl, iso-Butyl, Methoxy, Ethoxy, n-Propoxy, iso-Propoxy, Trifluormethyl, Difluormethoxy oder Trifluormethoxy.
- X: steht ganz besonders bevorzugt für Fluor, Chlor, Brom, Methyl, Ethyl, n-Propyl, iso-Propyl, n-Butyl, iso-Butyl, Methoxy, Ethoxy, n-Propoxy, iso-Propoxy, Trifluormethyl, Trifluormethoxy, Difluormethoxy, Cyano oder Nitro.
- Y: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Difluormethoxy, Cyano oder Nitro.
- Z: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Difluormethoxy, Cyano oder Nitro.
- A: steht ganz besonders bevorzugt für Methyl oder Ethyl.
- B: steht ganz besonders bevorzugt für Wasserstoff oder Methyl.
- G: steht ganz besonders bevorzugt für Wasserstoff (a) oder für einen der Reste 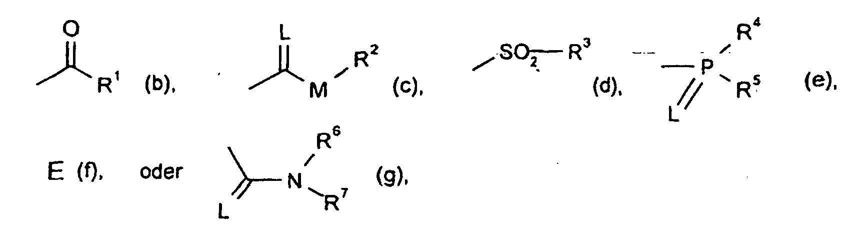 (insbesondere für einen der Reste (a), (b) oder (c)),
in welchen
E für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R3: steht ganz besonders bevorzugt für jeweils gegebenenfalls durch Fluor oder Chlor substituiertes Methyl, Ethyl, n-Propyl, iso-Propyl, n-Butyl, tert.-Butyl, oder für jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl, C₁-C₄-Alkoxy-C₂-C₄-alkyl oder für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Insbesondere bevorzugt sind Verbindungen der Formel (I), in denen A für CH₃ und B für Wasserstoff steht, insbesondere in Kombination mit den für G genannten, ganz besonders bevorzugten Resten.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-a) genannt:

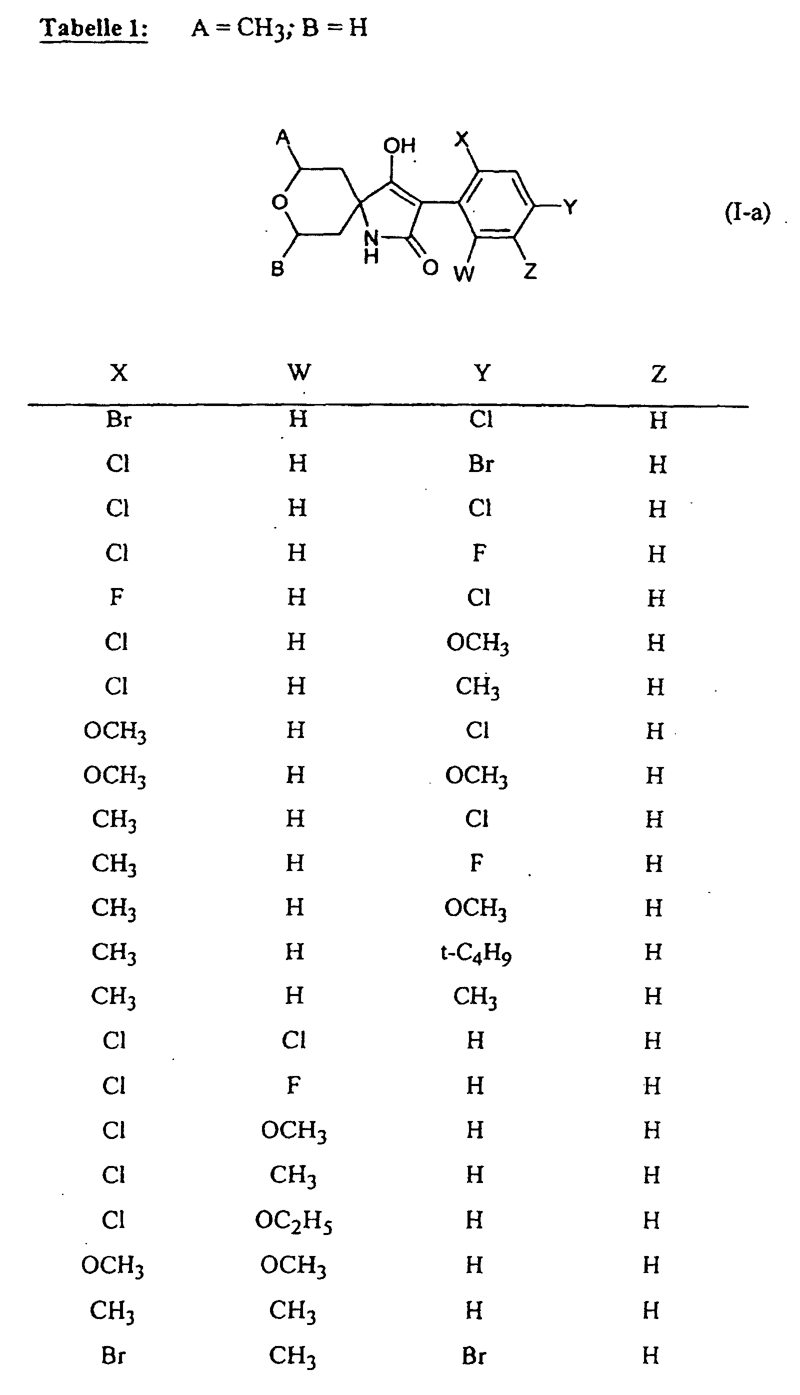

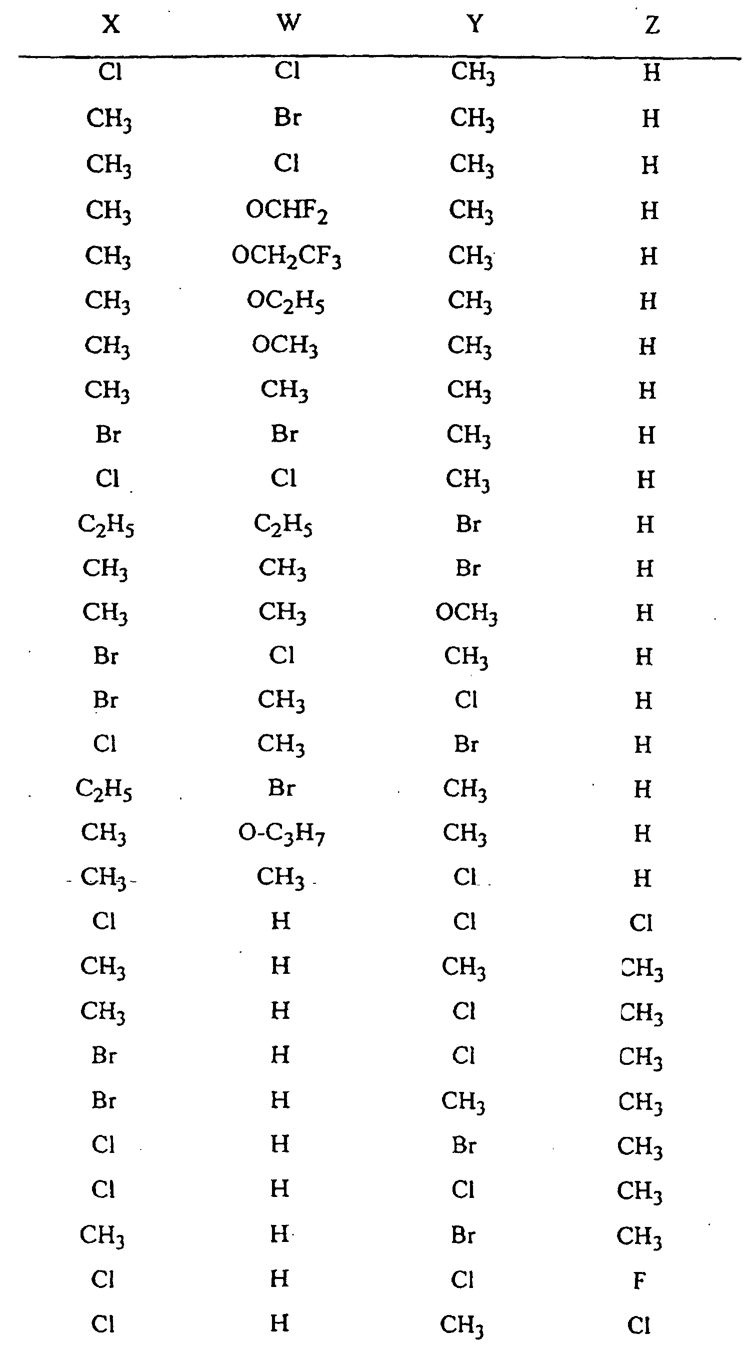

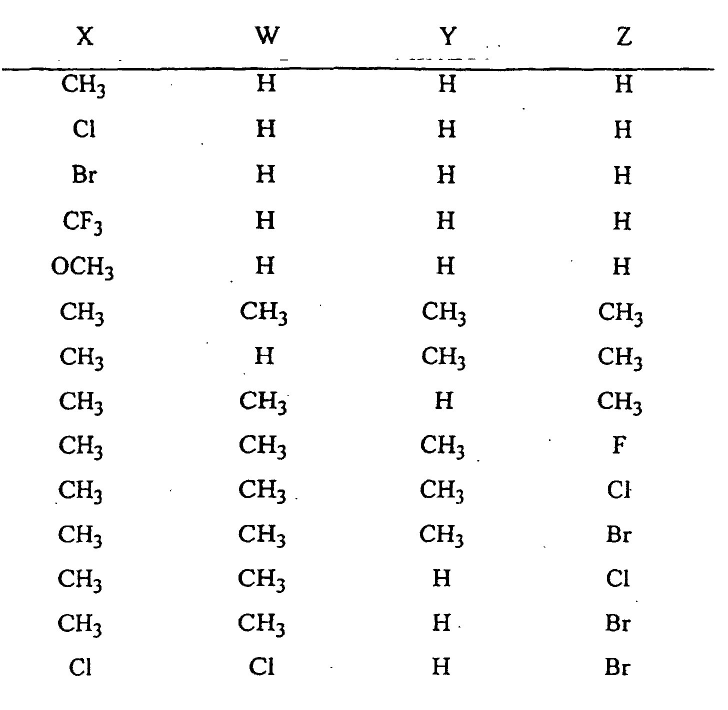

Verwendet man gemäß Verfahren (A) N-[(4-Chlor-2,6-dimethyl)-phenylacetyl]-4-amino-4-carboxyethyl-2-methyl-tetrahydropyran als Ausgangsstoff, so kann der Verlauf des erfindungsgemäßen Verfahrens durch folgendes Reaktionsschema wiedergegeben werden:

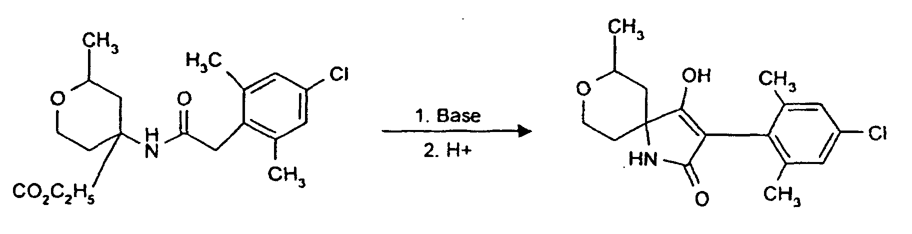

Verwendet man gemäß Verfahren (Bα) 3-[(2-Chlor-4-methyl)-phenyl]-5,5-[(2-ethyl)-ethylenoxyethyl]-pyrrolidin-2,4-dion und Pivaloylchlorid als Ausgangsstoffe, so kann der Verlauf des erfindungsgemäßen Verfahrens durch folgendes Reaktionsschema wiedergegeben werden:

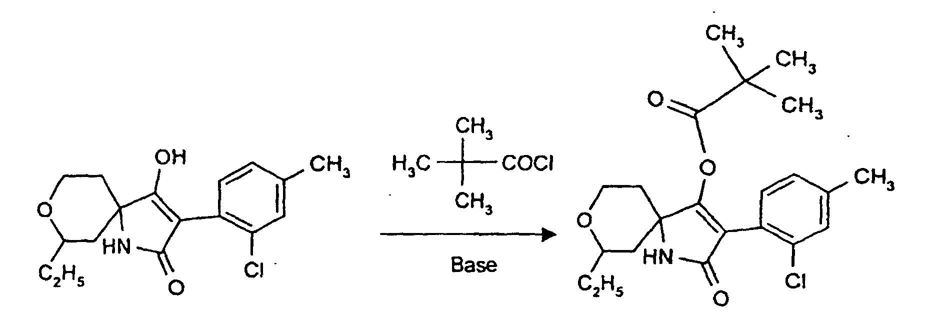

Verwendet man gemäß Verfahren (B) (Variante β) 3-[(2,4-Dichlor)-phenyl]-5,5-[(2-methyl)-ethylenoxyethyl]-pyrrolidin-2,4-dion und Acetanhydrid als Ausgangsverbindungen, so kann der Verlauf des erfindungsgemäßen Verfahrens durch folgendes Reaktionsschema wiedergegeben werden:

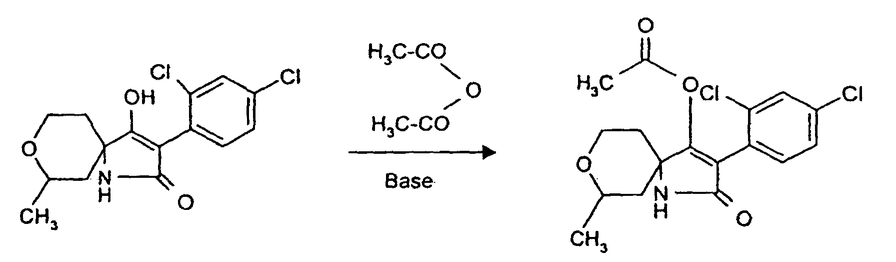

Verwendet man gemäß Verfahren (C) 8-[(2,4-Dichlor)-phenyl]-5,5-[(2-methyl)-ethylenoxyethyl]-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

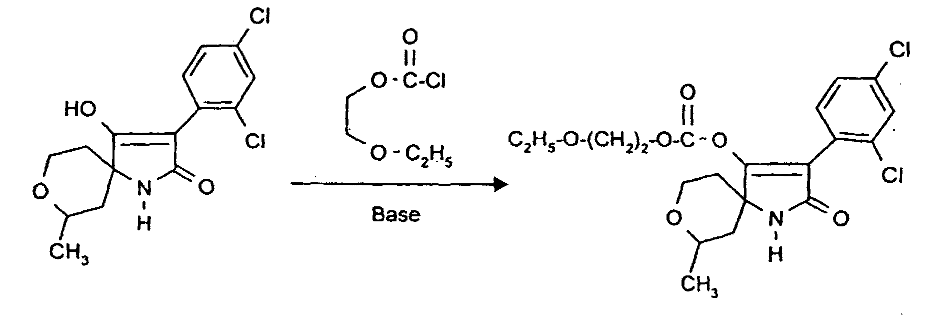

Verwendet man gemäß Verfahren (D) 3-[(2,6-Dibrom-4-methyl)-phenyl]-5,5-[(2-ethyl)-ethylenoxyethyl]-pyrrolidin-2,4-dion und Chlormonothioameisensäuremethylester als Ausgangsprodukte, so kann der Reaktionsverlauf folgendermaßen wiedergegeben werden:

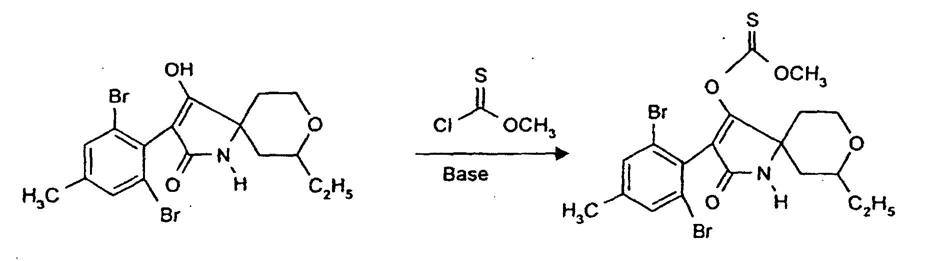

Verwendet man gemäß Verfahren (E) 2-[(2,4,6-Trimethyl)-phenyl]-5,5-[(2-methyl)-ethylenoxyethyl]-pyrrolidin-2,4-dion und Methansulfonsäurechlorid als Ausgangsprodukte, so kann der Reaktionsverlauf durch folgendes Reaktionsschema wiedergegeben werden:

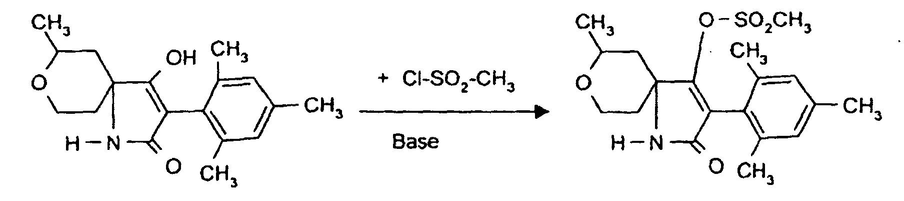

Verwendet man gemäß Verfahren (F) 2-[(4-Brom-2-chlor-6-methyl)-phenyl]-4-hydroxy-5,5-[(2-methyl)-ethylenoxyethyl]-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

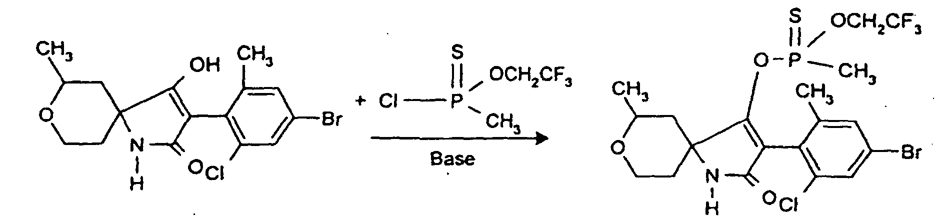

Verwendet man gemäß Verfahren (G) 3-[(2,4-Dichlor)-6-methylphenyl]-5,5-[(2-ethyl)-ethylenoxyethyl]-pyrrolidin-2,4-dion und NaOH als Komponenten, so kann der Verlauf des erfindungsgemäßen Verfahrens durch folgendes Reaktionsschema wiedergegeben werden:

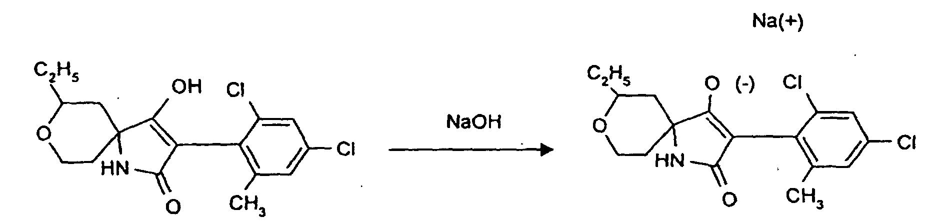

Verwendet man gemäß Verfahren (H) (Variante α) 3-[(2-Chlor-4-brom-5-methyl)-phenyl]-4-hydroxy-5,5-[(2-methyl)-ethylenoxyethyl]-pyrrolidin-2,4-dion und Ethylisocyanat als Ausgangsprodukte, so kann der Reaktionsverlauf durch folgendes Reaktionsschema wiedergegeben werden:

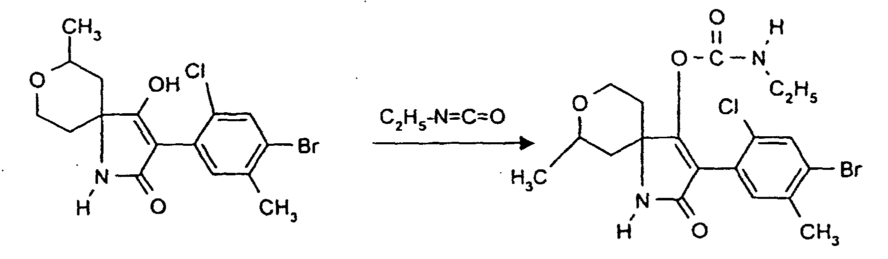

Verwendet man gemäß Verfahren (H) (Variante β) 3-[(2-Chlor-4,6-dimethyl)-phenyl]-5,5-[(2-methyl)-ethylenoxyethyl]-pyrrolidin-2,4-dion und Dimethylcarbamidsäurechlorid als Ausgangsprodukte, so kann der Reaktionsverlauf durch folgendes Schema wiedergegeben werden:

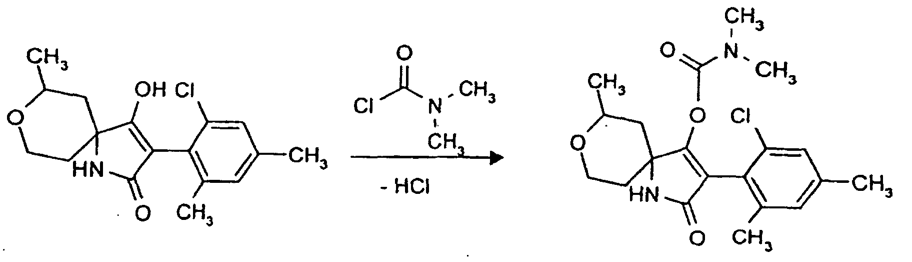

Die beim erfindungsgemäßen Verfahren (A) als Ausgangsstoffe benötigten Verbindungen der Formel (II) 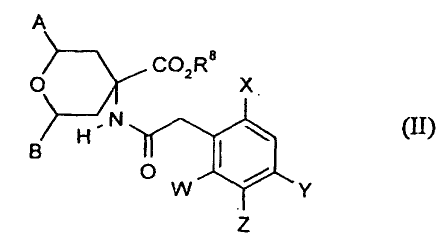 in welcher
- A, B, W, X, Y, Z und R⁸: die oben angegebenen Bedeutungen haben,
sind neu.

Man erhält die Acylaminosäureester der Formel (II) beispielsweise, wenn man Aminosäurederivate der Formel (XIII) 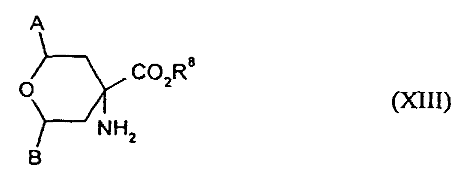 in welcher
- A, B und R⁸: die oben angegebene Bedeutung haben,
mit substituierten Phenylessigsäurehalogeniden der Formel (XIV) 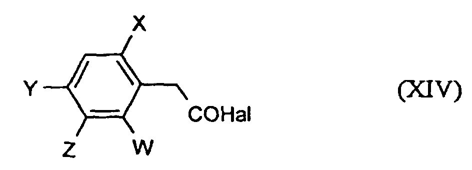 in welcher
W, X, Y und Z die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XV) 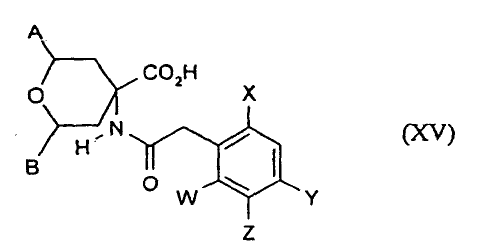 in welcher
A, B, W, X, Y und Z die oben angegebenen Bedeutungen haben,
verestert (Chem. Ind. (London) 1568 (1968)).

Die Verbindungen der Formel (XV) 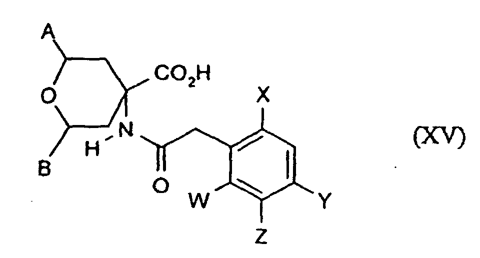 in welcher
A, B, W, X, Y und Z die oben angegebenen Bedeutungen haben,
sind neu.

Man erhält die Verbindungen der Formel (XV) beispielsweise, wenn man 4-Aminotetrahydropyran-4-carbonsäuren der Formel (XVI) 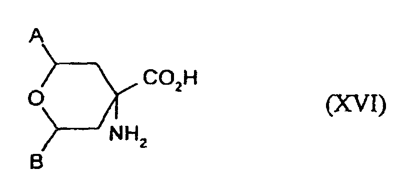 in welcher
- A und B: die oben genannten Bedeutungen haben,
mit substituierten Phenylessigsäurehalogeniden der Formel (XIV) 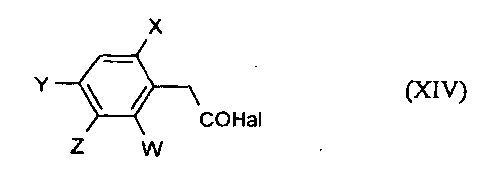 in welcher
W, X, Y und Z die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XIV) sind teilweise neu und lassen sich nach bekannten Verfahren herstellen (vgl. z.B. DE-196 49 665).

Man erhält die Verbindungen der Formel (XIV) beispielsweise, indem man substituierte Phenylessigsäuren der Formel (XVII) 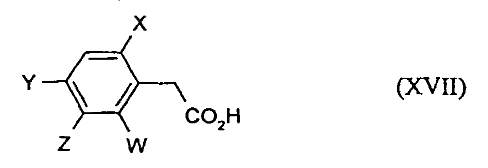 in welcher
- W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XVII) sind teilweise neu, sie lassen sich nach literaturbekannten Verfahren herstellen (Organikum 15. Auflage, S. 533, VEB Deutscher Verlag der Wissenschaften, Berlin 1977, vgl. z.B. DE-196 49 665).

Man erhält die Verbindungen der Formel (XVII) beispielsweise, indem man substituierte Phenylessigsäureester der Formel (XVIII) 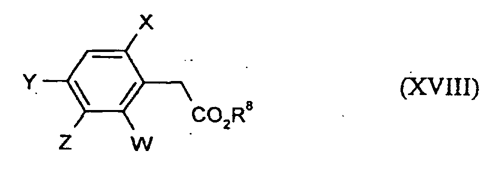 in welcher
- W, X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XVIII) sind teilweise neu, sie lassen sich nach im Prinzip bekannten Verfahren herstellen.

Man erhält die Verbindungen der Formel (XVIII) beispielsweise, indem man substituierte 1,1,1-Trichlor-2-phenylethane der Formel (XIX) 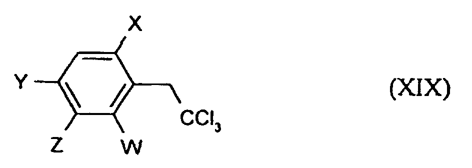 in welcher
- W, X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE-3 314 249).

Die Verbindungen der Formel (XIX) sind teilweise neu, sie lassen sich nach im Prinzip bekannten Verfahren herstellen.

Man erhält die Verbindungen der Formel (XIX) beispielsweise, wenn man Aniline der Formel (XX) 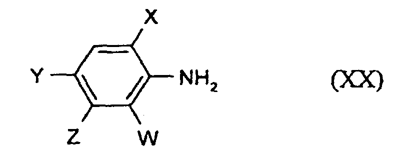 in welcher
- W, X, Y und Z: die oben angegebene Bedeutung haben,
in Gegenwart eines Alkylnitrits der Formel (XXI)

R¹³-ONO (XXI)

in welcher
- R¹³: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XX) sind teilweise bekannt. Sie lassen sich nach literaturbekannten Verfahren darstellen, beispielsweise durch Reduktion der entsprechenden Nitroverbindungen oder Halogenierung der Aniline bzw. Acetanilide und anschließende Rückspaltung.

Die Verbindungen der Formel (XXI) sind bekannte Verbindungen der Organischen Chemie. Kupfer(II)-chlorid und Vinylidenchlorid sind lange bekannt und käuflich.

Die substituierten cyclischen Aminocarbonsäuren der Formel (XVI) sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

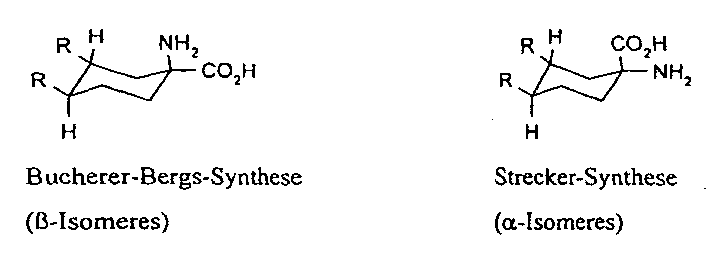

Die Verbindungen der Formel (XIII) und (XVI) sind neu. Sie lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975)).

Weiterhin lassen sich die bei dem obigen Verfahren (A) verwendeten Ausgangsstoffe der Formel (II) 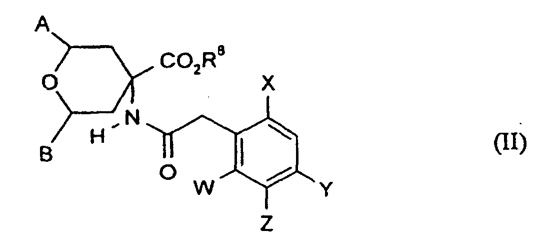 in welcher
A, B, W, X, Y, Z und R⁸ die oben angegebenen Bedeutungen haben,
herstellen, wenn man Aminonitrile der Formel (XXII) 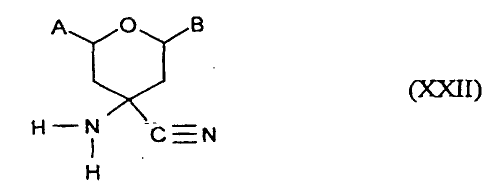 in welcher
- A und B: die oben angegebenen Bedeutungen haben,
mit substituierten Phenylessigsäurehalogeniden der Formel (XIV) 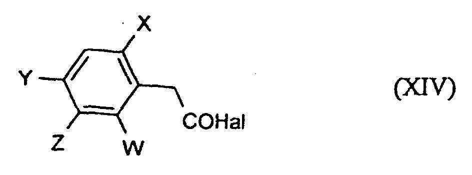 in welcher
W, X, Y, Z und Hal die oben angegebenen Bedeutungen haben,
zu Verbindungen der Formel (XXIII) 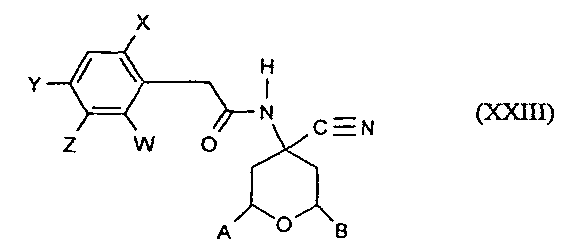 in welcher
A, B, W, X, Y und Z die oben angegebenen Bedeutungen haben,
umsetzt,
und diese anschließend einer sauren Alkoholyse unterwirft.

Die Verbindungen der Formel (XXIII) sind ebenfalls neu. Die Verbindungen der Formel (XXII) sind ebenfalls neu.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) sind allgemein bekannte Verbindungen der organischen bzw. anorganischen Chemie.

Die Verbindungen der Formeln (XIV), (XVII), (XVIII), (XIX) und (XX) sind darüber hinaus aus den eingangs zitierten Patentanmeldungen bekannt und/oder lassen sich nach den dort angegebenen Methoden herstellen (vgl. auch DE-A 196 49 665 und DE-A 196 13 171).

Das Verfahren (A) ist dadurch gekennzeichnet, daß man Verbindungen der Formel (II), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B_{α}) ist dadurch gekennzeichnet, daß man Verbindungen der Formel (I-a) mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (B_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (B_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (B_{α}) werden die Ausgangsstoffe der Formel (I-a) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (I-a) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Bβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (I-a) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß . vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (I-a) mit Chlorameisensäureestem oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (C) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (VII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (D) ist dadurch gekennzeichnet, daß man Verbindungen der Formel (I-a) mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen der Formel (I-a) dar, kann auf den weiteren Zusatz von Säurebindemitteln verzichtet werden.

Als Basen können beim Verfahren (D) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, daß man Verbindungen der Formel (I-a) jeweils mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (IX) bei -20 bis 150°C, vorzugsweise bei 0 bis 70°C um.

. Das Verfahren (E) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, daß man Verbindungen der Formeln (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen der Formel (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Das Verfahren (F) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgerührt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (G) ist dadurch gekennzeichnet, daß man Verbindungen der Formel (I-a) jeweils mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (H) ist dadurch gekennzeichnet, daß man Verbindungen der Formel (I-a) jeweils mit (Hα) Verbindungen der Formel (XI) gegebenenfalls in Gegenwart eines Verdünnungsmittels und gegebenenfalls in Gegenwart eines Katalysators oder (Hβ) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (Hα) setzt man pro Mol Ausgangsverbindung der Formel (I-a) ca. 1 Mol Isocyanat der Formel (XI) bei 0 bis 100°C, vorzugsweise bei 20 bis 50°C um.

Das Verfahren (Hα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Hβ) setzt man pro Mol Ausgangsverbindung der Formel (I-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp. ,
Aus der Ordnung der Thysanoptera z.B. Frankliniella occidentalis, Hercinothrips femoralis, Thrips palmi, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Liriomyza spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit nach Blatt- und Bodenanwendung aus.

Sie lassen sich mit besonders gutem Erfolg zur Bekämpfung von pflanzenschädigenden Insekten einsetzen, wie beispielsweise gegen die Larven des Meerrettichblattkäfers (Phaedon cochleariae), gegen die Larven der grünen Reiszikade (Nephotettix cincticeps) und gegen die Larven der grünen Pfirsichblattlaus (Myzus persicae).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und daneben bevorzugt Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb,
   Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron,
   Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Hamstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise - Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares orga-nisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributyl- phosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, - 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Beispiel (I-a-1)

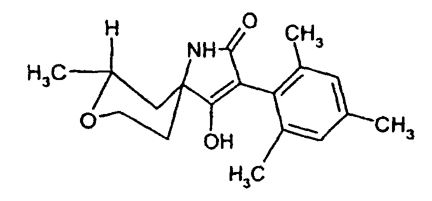

Zu 18,4 g (0,16 mol) Kalium-tert.-butylat in 63 ml wasserfreiem Tetrahydrofuran (THF) tropft man bei Rückflußtemperatur 24,8 g der Verbindung gemäß Beispiel (II-2) in 150 ml wasserfreiem Toluol und rührt noch 1,5 Stunden unter Rückfluß. Dann versetzt man mit 240 ml Wasser, trennt die Phasen und extrahiert die Toluolphase mit Wasser. Die vereinigten wäßrigen Phasen werden mit Toluol gewaschen und bei 10 bis 20°C mit ca. 26 ml konz. Salzsäure angesäuert. Man saugt ausgefallenen Feststoff ab, wäscht und trocknet. Zur Reinigung wird in einem Gemisch aus Methyltert.-butylether (MTB-Ether) und n-Hexan verrührt.

Ausbeute 15,6 g (69 % der Theorie), Fp.: >220°C.

Analog bzw. gemäß den allgemeinen Angaben zur Herstellung erhält man die folgenden Verbindungen der Formel (I-a):

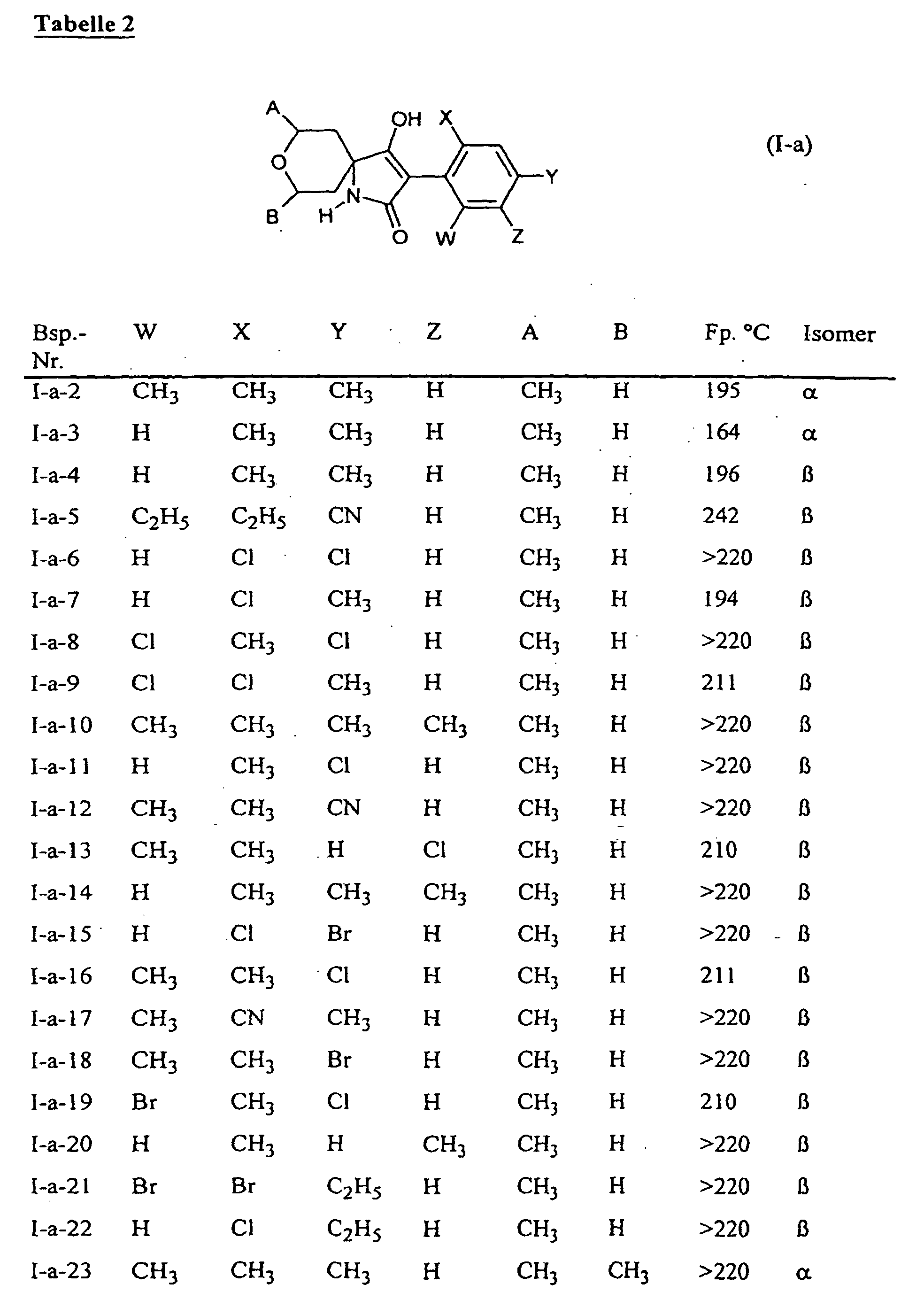

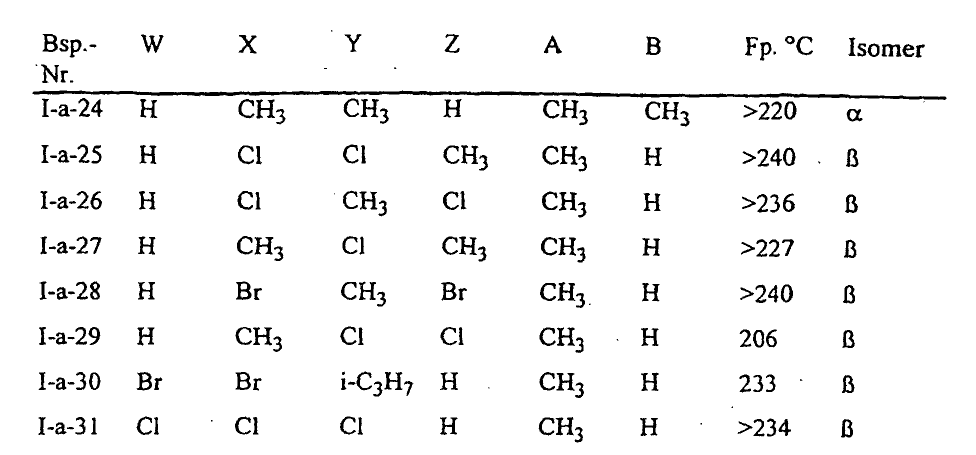

### Beispiel (I-b-1)

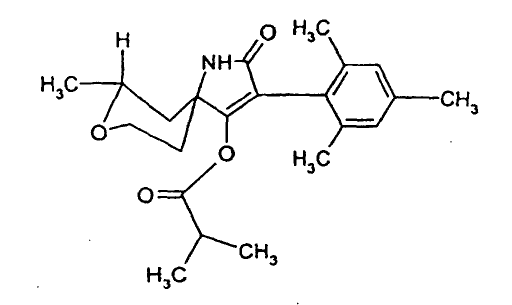

Zu 3,62 g der Verbindung gemäß Beispiel (I-a-1) in 70 ml wasserfreiem Methylenchlorid gibt man 2,52 mol (18 mmol) Triethylamin. Bei 0 bis 10°C tropft man zu diesem Gemisch 1,9 ml (18 mmol) Isobuttersäurechlorid in 5 ml wasserfreiem Methylenchlorid und rührt bei Raumtemperatur bis die Reaktion beendet ist. Dann wäscht man 2 mal mit je 50 ml 0,5 N NaOH, trocknet und engt ein. Der Rückstand wird aus MTB-Ether/n-Hexan umkristallisiert.

Ausbeute 1,6 g (35 % der Theorie), Fp.: 209°C.

Analog bzw. gemäß den allgemeinen Angaben zur Herstellung erhält man die folgenden Verbindungen der Formel (I-b):

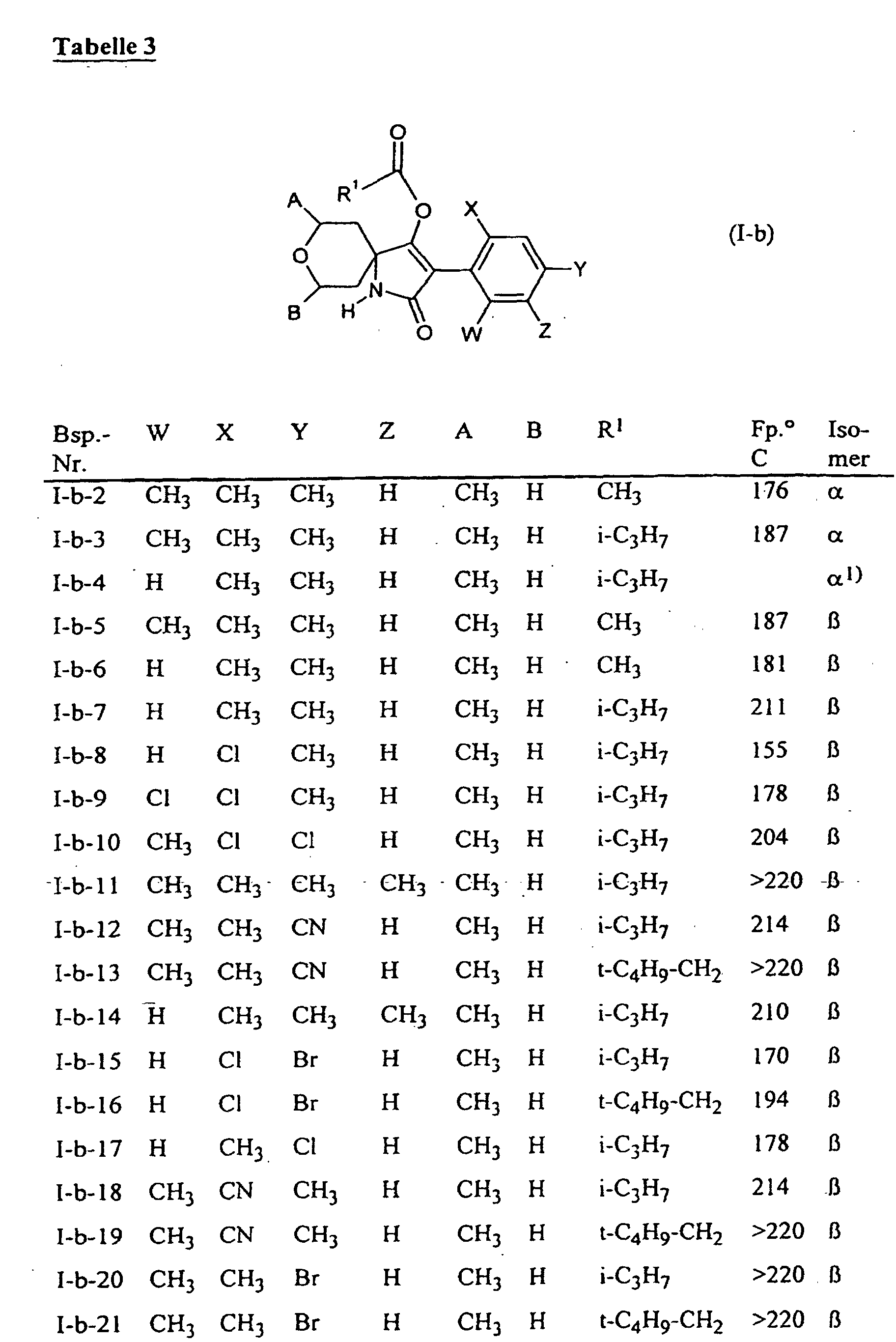

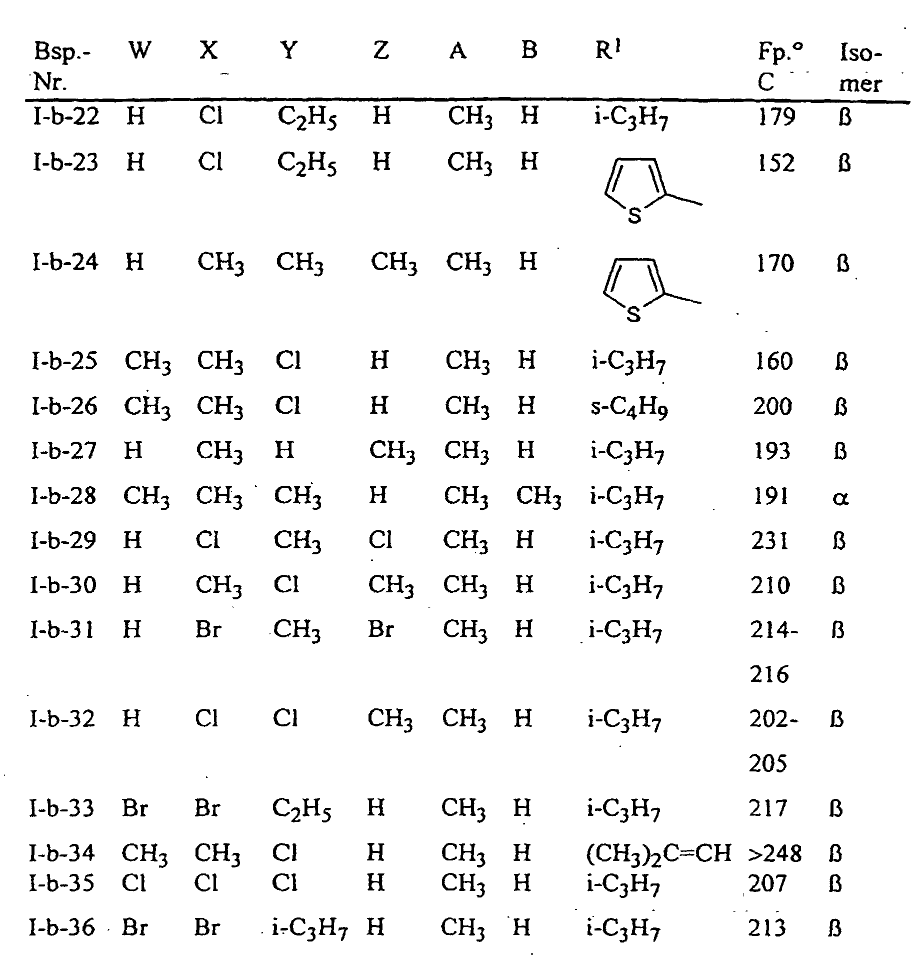

¹⁾¹H-NMR (200 MHz, CDCl₃): δ = 1,0-1,05 (4s, 6H, CH(CH₃)₂), 2.25, 2.28 (25, 6H, ArCH₃).

### Beispiel (I-c-1)

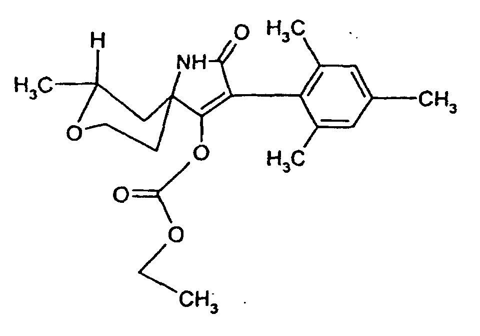

Zu 3,62 g der Verbindung gemäß Beispiel (I-a-1) und 1,7 ml (12 mmol) Triethylamin in 70 ml wasserfreiem Methylenchlorid tropft man bei 0 bis 10°C 1,2 ml (12 mmol) Chlorameisensäureethylester in 5 ml wasserfreiem Methylenchlorid und rührt bei Raumtemperatur bis die Reaktion beendet ist. Dann wäscht man 2 mal mit je 50 ml 0,5 N NaOH, trocknet, engt ein und kristallisiert den Rückstand aus MTB-Ether/n-Hexan um.

Ausbeute 2,70 g (60 % der Theorie), Fp.: 217°C.

Analog bzw. gemäß den allgemeinen Angaben zur Herstellung erhält man die folgenden Verbindungen der Formel (I-c):

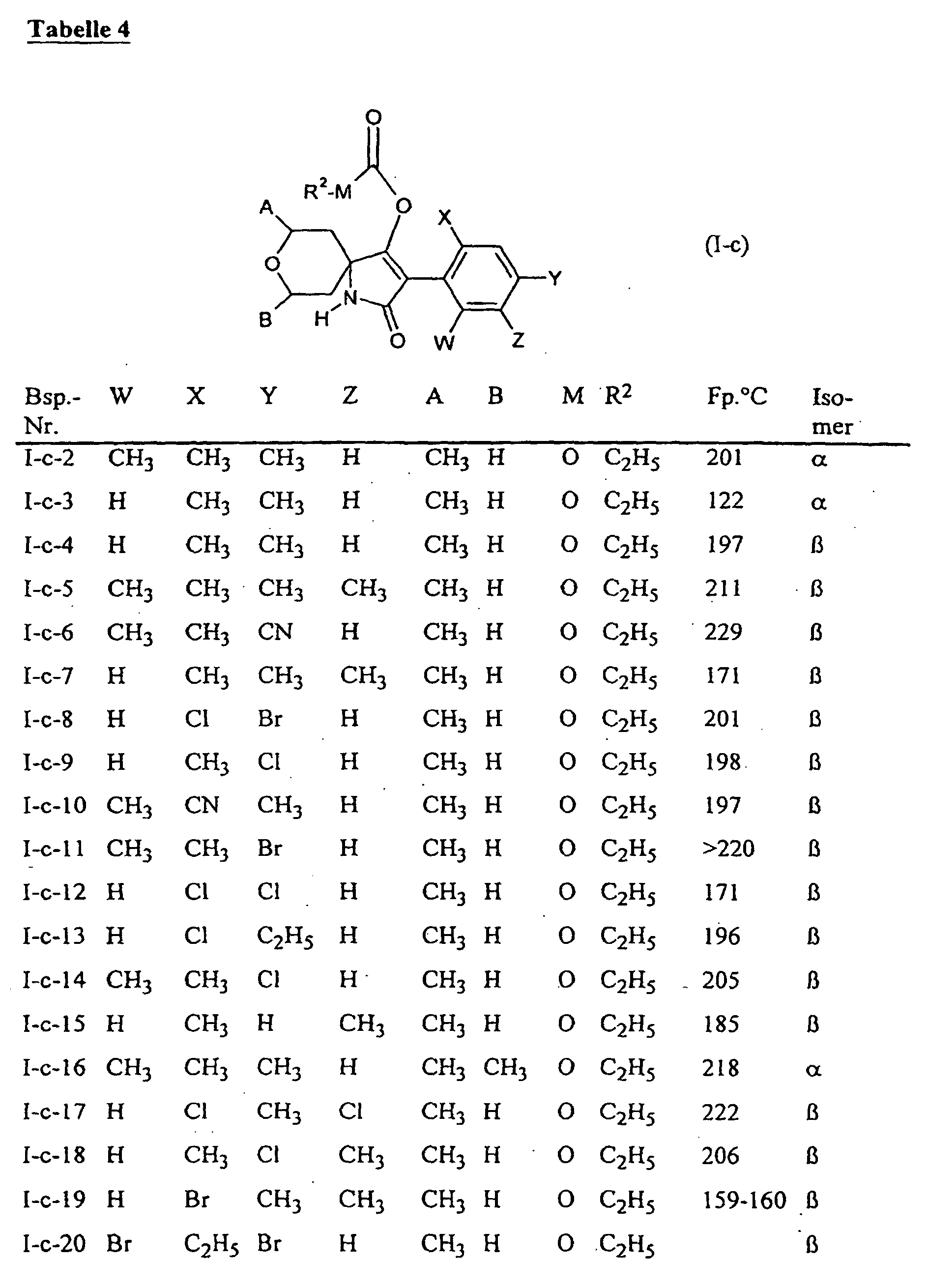

### Beispiel (II-1)

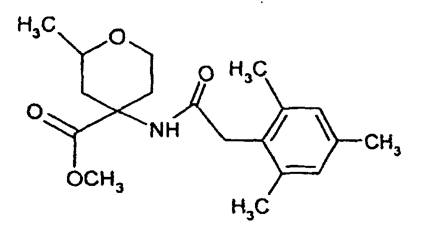

Bei 30 bis 40°C tropft man 27,8 g der Verbindung gemäß Beispiel (XXIII-1) in 180 ml Methylenchlorid zu 45,4 g konzentrierter Schwefelsäure und rührt noch 2 Stunden bei 30 bis 40°C. Dann tropft man 64 ml wasserfreies Methanol zu und rührt noch 6 Stunden bei 40 bis 70°C. Dann gibt man auf 0,46 kg Eis, extrahiert mit Methylenchlorid, wäscht mit wäßriger NaHCO₃-Lösung, trocknet und engt ein. Der Rückstand wird aus MTB-Ether/n-Hexan umkristallisiert.
Ausbeute 19,80 g (64 % der Theorie), Fp.: 101°C.

### Beispiel (II-2)

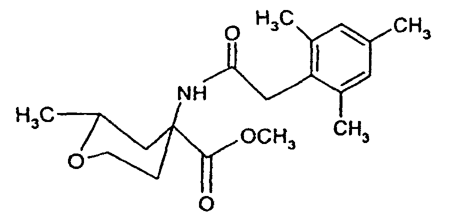

Zu 20,98 g der Verbindung gemäß Beispiel (XIII-1) und 30,8 ml (0,22 mol) Triethylamin in 200 ml wasserfreiem THF tropft man bei 0 bis 10°C 19,6 g Mesitylenessigsäurechlorid in 20 ml wasserfreiem THF und rührt bei Raumtemperatur bis die Reaktion beendet ist. Man saugt ab, wäscht nach und engt ein. Der Rückstand wird in Methylenchlorid aufgenommen, mit 200 ml 1 N HCl gewaschen, getrocknet und eingeengt. Nach Säulenchromatographie an Kieselgel mit Cyclohexan/Essigester 2/1 erhält man 24,0 g (72 % der Theorie). ¹H-NMR (200 Mhz, CDCl₃): δ = 1,12 (d, 3H, CH-CH₃), 3,51 (α), 3,6 (β) (2s, 2H, CH₂, CONH, β/α ca. 3:1), 3,71 , 3,75 (α/β) (2s, 3H, CO₂CH₃, (β/α 3:1), 6,90 (α), 6,92 (β) (2s, 2H, ArH).

Analog bzw. gemäß den allgemeinen Angaben zur Herstellung erhält man die folgenden Verbindungen der Formel (II):

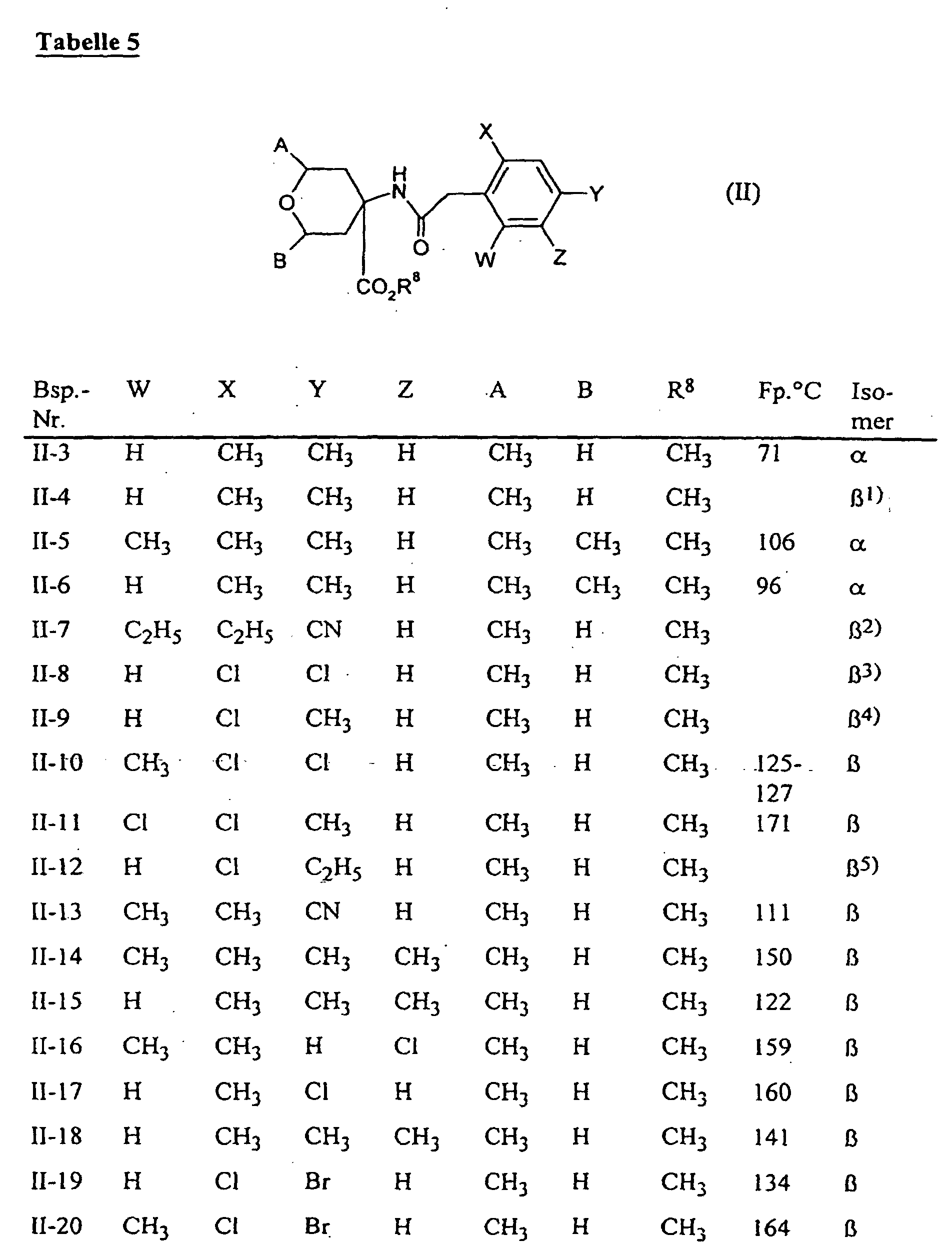

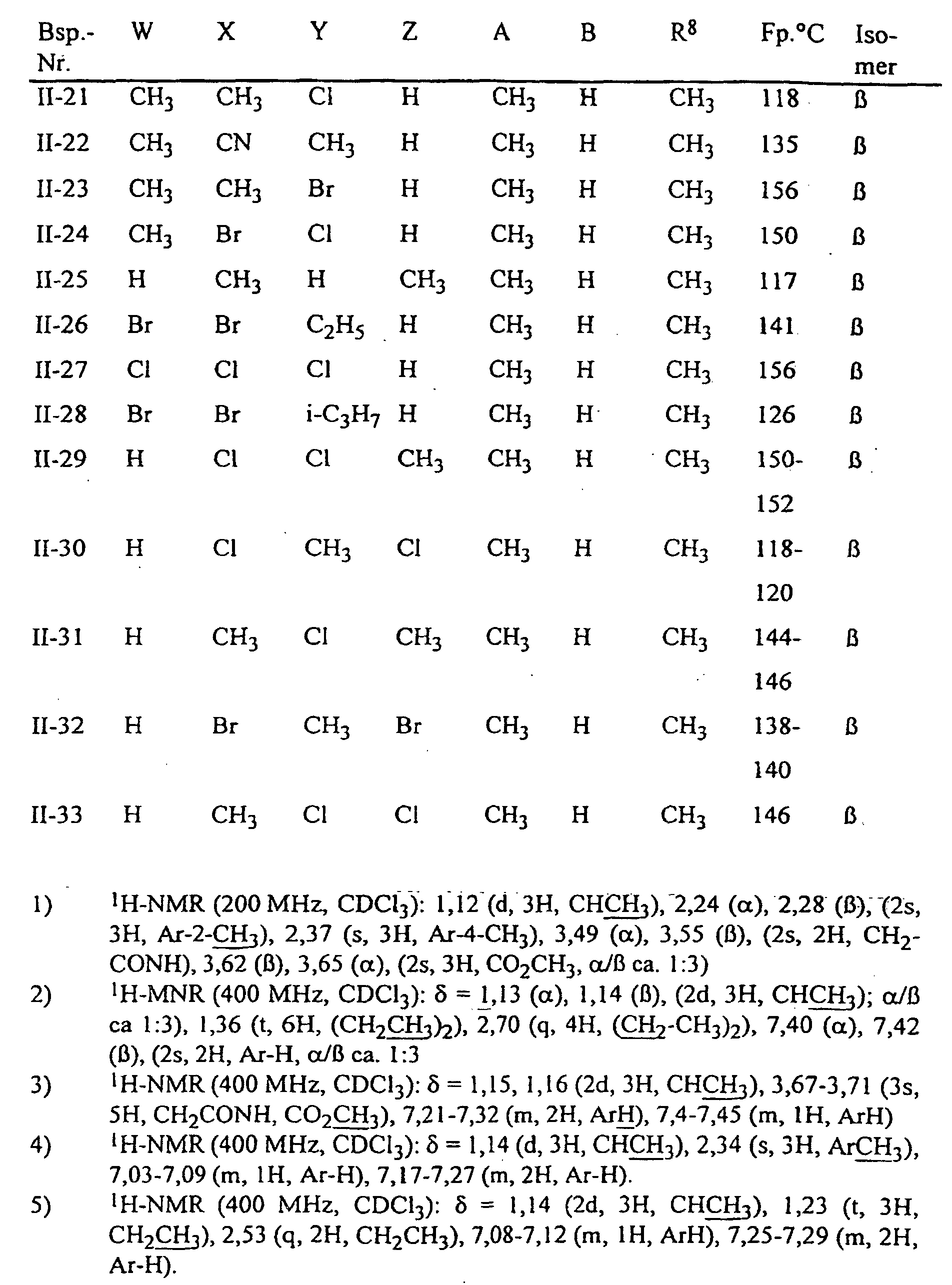

### Beispiel (XIII-1)

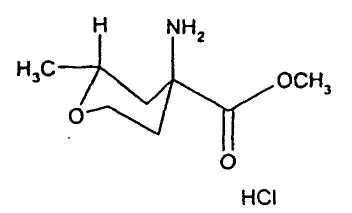

Zu 92,3 g der Verbindung gemäß Beispiel (XVI-1) in 870 ml wasserfreiem Methanol tropft man bei 0 bis 5°C 73,2 ml (0,87 mol) Thionylchlorid, rührt 30 Minuten bei ca. 0°C und über Nacht bei ca. 40°C. Man filtriert, engt ein, verrührt mit etwas MTB-Ether, saugt ab, wäscht nach und trocknet.

Ausbeute 87,0 g (86 % der Theorie), Fp.:>220°C.

### Beispiel (XVI-1)

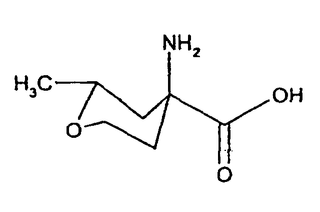

122,8 g der Verbindung gemäß nachstehendem Beispiel und 130 g NaOH in 2,5 l Wasser werden im Autoklaven 2 Stunden auf 195°C erhitzt, wobei der Druck auf ca. 20 bar ansteigt. Dann engt man auf ca. ¹/₃ desVolumens ein, gibt bei 0 bis 10°C konzentrierte HCl zu, bis der pH-Wert 5 bis 6 beträgt, engt ein, kocht mit Methanol auf, saugt ab und engt das Filtrat ein.

Ausbeute 92,3 g (86 % der Theorie), Fp.: >220°C.

### Beispiel : Verbindung der Formel

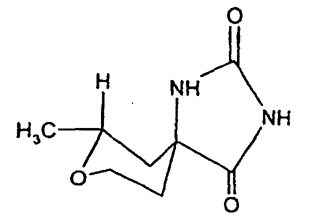

Im Autoklaven rührt man ein Gemisch bestehend aus 76 g 3-Methyl-tetrahydropyran-4-on, 71,9 g (1,468 mol) Natriumcyanid, 96 g (1 mol) Ammoniumcarbonat, 1,321 konzentrierte Ammoniaklösung und 1,321 Ethanol 3 Stunden bei 120°C, wobei der Innendruck auf ca. 60 bar ansteigt (vor dem Aufheizen gibt man ²/₃ des gewünschten Reaktionsdruckes vor). Man engt ein, trocknet mit Toluol, kocht mit Methanol auf und saugt ab. Die Mutterlauge wird auf 400 ml eingeengt und durch Zugabe von MTB-Ether wird weiterer Feststoff ausgefällt und abgesaugt. Die Mutterlauge wird eingeengt. Die vereinigten Rückstände werden in Ethanol aufgekocht, man saugt ab (A) und engt das Filtrat ein (B). (A) und (B) werden vereinigt.
Ausbeute 126,0 g (100 % der Theorie).

### Beispiel (XXIII-1)

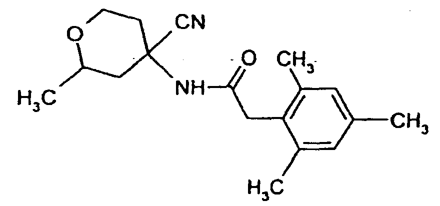

Zu 15,6 g der Verbindung gemäß Beispiel (XXII-1) und 15,4 ml Triethylamin in 220 ml wasserfreiem THF tropft man bei 0 bis 10°C 21,6 g Mesitylenessigsäurechlorid in 20 ml wasserfreiem THF und rührt bei Raumtemperatur, bis die Reaktion beendet ist. Man rührt in 0,6 1 Eiswasser und 0,2 1 1 N HCl ein, saugt ab und nimmt den Rückstand in Methylenchlorid auf. Man trocknet, engt ein und kristallisiert aus MTB-Ether/n-Hexan um.
Ausbeute 27,8 g (84 % der Theorie), Fp.: 121°C.

Analog bzw. gemäß den allgemeinen Angaben zur Herstellung erhält man die folgenden Verbindungen der Formel (XXIII):

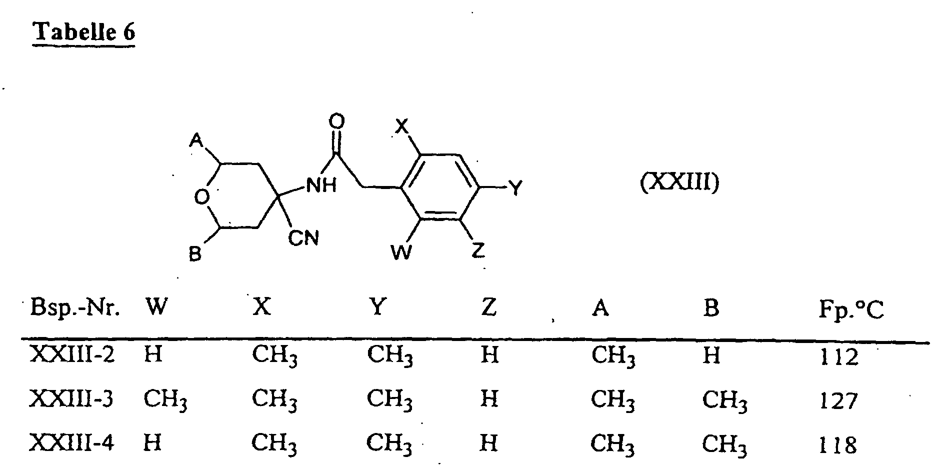

### Beispiel (XIV-1)

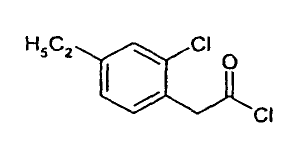

99,3 g 2-Chlor-4-ethylphenylessigsäure und 109 ml (1,5 mol) Thionylchlorid werden bei 70°C bis zum Ende der Gasentwicklung gerührt. Bei 50°C wird überschüssiges Thionylchlorid im Vakuum entfernt. Der Rückstand wird destilliert.

Ausbeute 99,10 g (91 % der Theorie), Kp. 121°C/0-35 mbar.

### Beispiel (XVII-1)

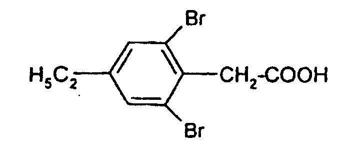

102,5 g (16,9 %ig, 0,05 mol) der Verbindung gemäß Beispiel (XVIII-1), 14,1 g KOH, 17,8 ml Wasser und 35,5 ml Methanol werden zusammen 5 Stunden unter Rückfluß erhitzt. Dann engt man ein und nimmt den Rückstand in Wasser auf. Man wäscht mit Essigester und säuert die wäßrige Phase mit konz. HCl an (pH 1). Man saugt ab und trocknet.

Ausbeute 14,4 g (80,6 % der Theorie), Fp.: 140-142°C.

Analog bzw. gemäß den allgemeinen Angaben zur Herstellung erhält man folgende Verbindungen der Formel (XVII):

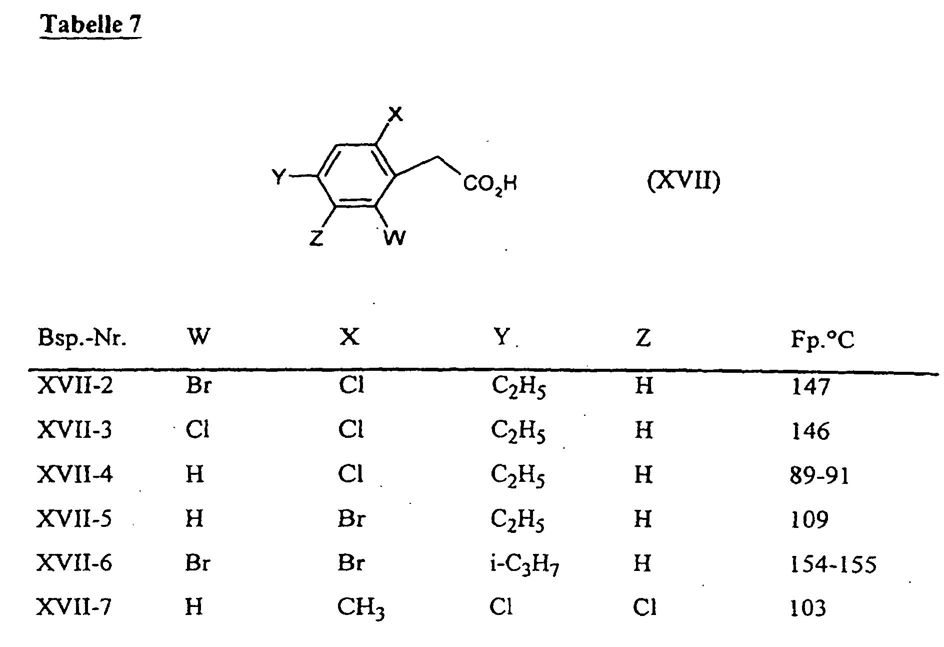

### Beispiel (XVIII-1)

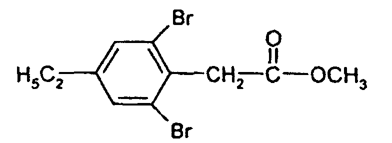

Zu 5 g (94,4 %ig, 0,0119 mol) der Verbindung gemäß Beispiel (XIX-1) in 5 ml Methanol tropft man unter Kühlung 9,1 ml 30 %iges Natriummethylat und rührt 5 Stunden unter Rückfluß. Nach dem Abkühlen tropft man 0,01 ml konzentrierte Schwefelsäure zu und rührt 1 Stunde unter Rückfluß. Dann wird eingeengt und der Rückstand in Wasser aufgenommen. Man extrahiert mit Methylenchlorid, trocknet und engt ein.

Ausbeute 1,80 g (43 % der Theorie), Öl.

Analog bzw. gemäß den allgemeinen Angaben zur Herstellung erhält man folgende Verbindungen der Formel (XVIII):

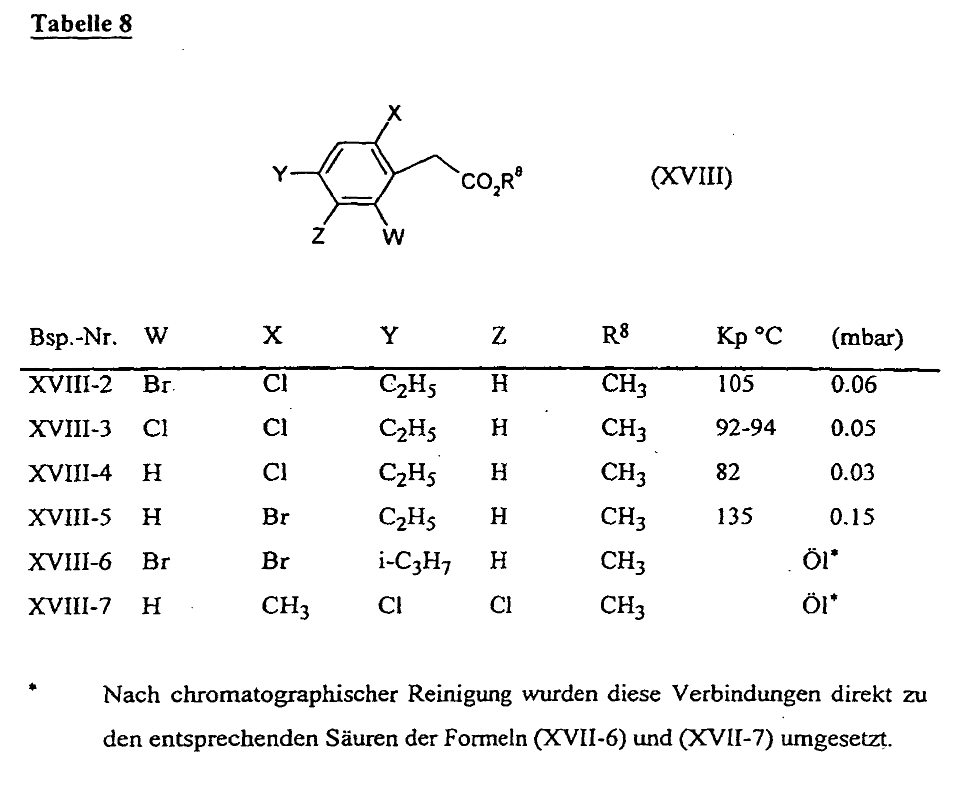

### Beispiel (XIX-1)

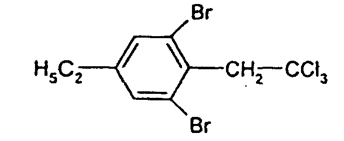

Zu 208 ml (1,746 mol) Butylnitrit in 684 ml wasserfreiem Acetonitril tropft man 1400 ml (17,4 mol) 1,1-Dichlorethan und dann 320 g (1,147 mol) der Verbindung gemäß Beispiel (XX-1) in 342 ml wasserfreiem Acetonitril. Man rührt über Nacht bei Raumtemperatur und gießt dann auf 4,6 1 20 %ige HCl. Man extrahiert mit MTB-Ether, wäscht die organische Phase mit 2 1 Wasser, trocknet und engt ein.
Ausbeute 434 g. Das Rohprodukt wird ohne weitere Reinigung weiter umgesetzt.

Analog bzw. gemäß den allgemeinen Angaben zur Herstellung erhält man folgende Verbindungen der Formel (XIX):

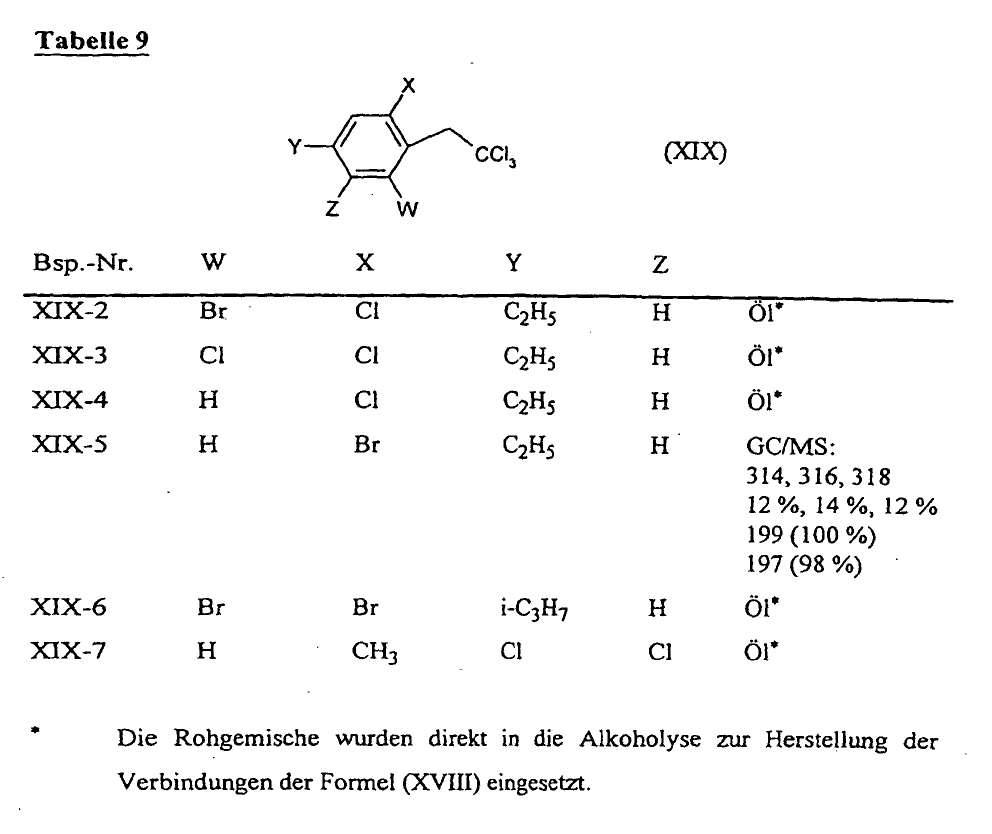

### Beispiel (XX-1)

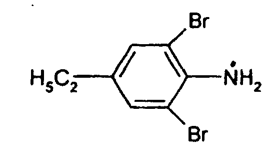

Zu 150 g (1,24 mol) 4-Ethylanilin in 1990 ml Eisessig tropft man bei 10-30°C 397 g (2,48 mol) Brom in 744 ml Eisessig und rührt noch 3 Stunden bei 30°C. Dann verdünnt man mit Wasser und stellt mit 25 %iger Ammoniaklösung alkalisch. Man saugt ab, nimmt in Methylenchlorid auf; trocknet und engt ein.

Ausbeute 320,0 g (93 % der Theorie), Fp.: 74°C.

### Beispiel (XX-2)

Analog zu Beispiel (XX-1) erhält man die Verbindung der Formel

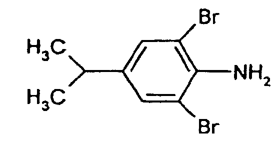

Fp.: 48°C.

### Beispiel (XXII-1)

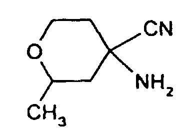

Zum Gemisch bestehend aus 50,9 g (0,75 mol) 25 %iger Ammoniaklösung, 17,2 g (0,32 mol) Ammoniumchlorid und 15,7 g (0,32 mol) Natriumcyanid in 48 ml Wasser tropft man bei Raumtemperatur 30,5 g (0,27 mol) 2-Methyl-tetrahydropyran-4-on (Herstellung siehe weiter unten) und rührt über Nacht bei 45°C. Nach üblicher Aufarbeitung erhält man 29,1 g (77 % der Theorie) Endprodukt als Öl.

### Beispiel (XXII-2)

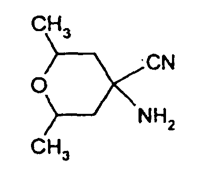

Diese Verbindung erhält man in analoger Weise als braunes Öl.

### Beispiel: Verbindung der Formel

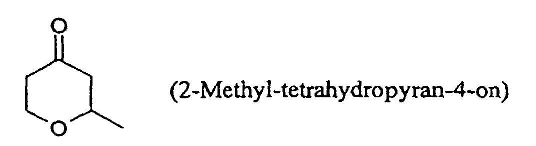

Zu 552,72 g (3,54 mol) NaH₂PO₄ x 2 H₂O und 179,7 g (1,55 mol) 85 %iger o-Phosphorsäure in 5500 ml Wasser tropft man bei einer Temperatur von ca. 100°C innerhalb von ca. 75 Minuten 364,37 g der Verbindung mit der Formel ClCH₂CH₂COCH₂CHClCH₃ (Herstellung siehe folgendes Beispiel) und rührt noch 8 Stunden bei 100°C.

Es wird auf ca. 0°C abgekühlt und 10 molare NaOH zugetropft, bis ein pH-Wert von 5 bis 6 erreicht ist. Nach Zugabe von 1500 ml Methylenchlorid wird das entstandene Salz abgesaugt und die wäßrige Phase 3 x mit je 1000 ml Methylenchlorid extrahiert. Man trocknet, engt ein und destilliert.

Ausbeute: 119,6 g (55 % der Theorie), Kp.: 62°C/15 mbar.

### Beispiel: Verbindung der Formel ClCH₂CH₂COCH₂CHClCH₃

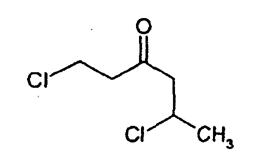

Zu 758,08 g (5,6 mol) AlCl₃ in 560 ml Methylenchlorid tropft man bei Raumtemperatur innerhalb von 15 Minuten 507,88 g 3-Chlorpropionylchlorid und leitet bei ca. 28 bis 30°C innerhalb von ca. 3 Stunden 189 g (4,5 mol) Propylen ein.

Vom überschüssigen AlCl₃ wird abdekantiert und das Reaktionsgemisch bei 0 bis 10°C langsam in ein Gemisch aus 508 ml Methylenchlorid und 2032 ml 1 N HCl getropft.

Die organische Phase wird abgetrennt, 3 mal mit je 500 ml Wasser gewaschen, getrocknet und eingeengt.

Ausbeute: 470 g (70 % der Theorie).

### Anwendungsbeispiele

### Beispiel A

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit werden die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Beispielen I-a-9, I-a-10, I-a-12, I-a-14, I-a-15, I-a-16, I-c-8, I-b-17, I-a-18, I-a-19, I-a-20, I-a-21, I-c-11 und I-c-12 bei einer beispielhaften Wirkstoffkonzentration von 0,1 % jeweils eine Abtötung von 100 % nach 6 Tagen.

### Beispiel B

Nephotettix-Test
- Lösungsmittel:: 20 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Beispielen I-b-2, I-b-4, I-c-3, I-b-5, I-b-1, I-c-1, I-b-6, I-b-7, I-c-4, I-a-7, I-b-8, I-b-11, I-c-5, I-c-7, I-b-17, I-c-11, I-a-22, I-b-22, I-b-23 und I-b-24 bei einer beispielhaften Wirkstoffkonzentration von 0,1 % jeweils eine Abtötung von 100 % nach 6 Tagen.

### Beispiel C

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test bewirkten z.B. die Verbindungen gemäß den Beispielen I-b-8, I-b-22, I-b-23, I-c-13, I-b-4, I-c-3, I-a-7 und I-a-8 bei einer beispielhaften Wirkstoffkonzentration von 0,1 % jeweils eine Abtötung von 100 % nach 7 Tagen.

### Beispiel D

### Spodoptera frugiperda-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Beispielen I-c-3, I-a-14, I-c-15, I-a-20, I-c-13 und I-b-24 bei einer beispielhaften Wirkstoffkonzentration von 0,1 % jeweils eine Abtötung von 100 % nach 7 Tagen.

### Beispiel E

### Tetranychus-Test (OP-resistent/Tauchbehandlung)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Beispielen I-b-2, I-b-3, 1-b-4, I-c-3, I-a-4, I-c-1, I-b-7, I-c-4, I-a-7, I-a-9 und I-b-8 bei einer beispielhaften Wirkstoffkonzentration von 0,1 % jeweils eine Wirkung von 100 % nach 14 Tagen, die Verbindungen gemäß den Beispielen I-b-15, I-b-16, I-b-17, I-b-22 und I-c-13 hatten diese Wirkung bei einer beispielhaften Wirkstoffkonzentration von 0,02 %.

### Beispiel F

### Grenzkonzentrations-Test / Wurzelsystemische Wirkung

- Testinsekt:: **Aphis fabae**
- Lösungsmittel:: 4 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit vorgekeimten Dicken Bohnen. Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den oben genannten Testtieren besetzt. Nach weiteren 6 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test hatten z.B. die Verbindungen gemäß den Beispielen I-a-1, I-b-4, I-a-4, I-b-5, I-a-16, I-b-1, I-b-3, I-c-1, I-c-2, I-b-17, I-b-10, I-a-16 und I-b-25 bei einer beispielhaften Wirkstoffkonzentration von 20 ppm eine Wirkung von jeweils 100 %.

### Beispiel G

### Grenzkonzentration / Wurzelsystemische Wirkung

- Testinsekt:: **Myzus persicae**
- Lösungsmittel:: 4 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Paprika, die sich im Keimblattstadium befinden. Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den oben genannten Testtieren besetzt. Nach weiteren 6 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test hatten z.B. die Verbindungen gemäß den Beispielen I-b-10, I-a-16 und I-b-25 bei einer beispielhaften Wirkstoffkonzentration von 20 ppm eine Wirkung von jeweils 100 %.

## Patentansprüche

1. Verbindungen der Formel (I) 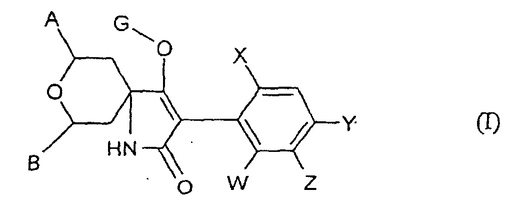 in welcher
W für Wasserstoff, Nitro, Cyano, Halogen, C₁-C₆-Alkyl, C₂-C₆-Alkenyl, C₂-C₆-Alkinyl, C₁-C₆-Alkoxy, C₁-C₄-Halogenalkyl oder C₁-C₄-Halogenalkoxy steht,
X für Halogen, C₁-C₆-Alkyl, C₂-C₆-Alkenyl, C₂-C₆-Alkinyl, C₁-C₆-Alkoxy, C₃-C₆-Alkenyloxy, C₁-C₄-Halogenalkyl, C₁-C₄-Halogenalkoxy, C₂-C₆-Halogenalkenyloxy, Cyano, Nitro oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
Y für Wasserstoff, Halogen, C₁-C₆-Alkyl, C₁-C₆-Alkoxy, C₁-C₄-Halogenalkyl, C₁-C₄-Halogenalkoxy, Cyano oder Nitro steht,
Z für Wasserstoff, Halogen, C₁-C₆-Alkyl, C₁-C₆-Alkoxy, C₁-C₄-Halogenalkyl, C₁-C₄-Halogenalkoxy, Hydroxy, Cyano, Nitro oder für jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht,
A für C₁-C₆-Alkyl steht,
B für Wasserstoff oder C₁-C₆-Alkyl steht,
G für Wasserstoff (a) oder für einen der Reste 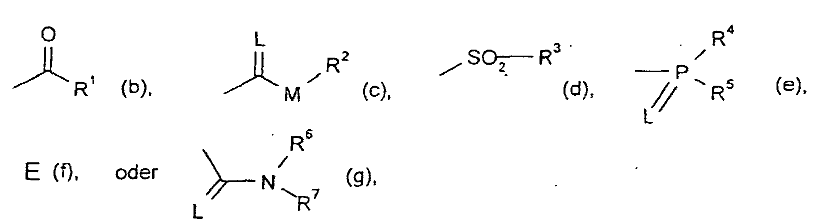 steht, in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff ,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di- (C₁-C₈-alkyl)amino, C₁-C₈-Alkylthio oder C₃-C₈-Alkenylthio oder für jeweils gegebenenfalls durch Halogen, Nitro; Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl, C₁-C₈-Alkoxy-C₂-C₈-alkyl oder für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

2. Verbindungen der Formel (I) gemäß Anspruch 1, in welcher
W für Wasserstoff, Nitro, Cyano, Fluor, Chlor, Brom, C₁-C₄-Alkyl, C₁-C₄-Alkoxy, C₁-C₂-Halogenalkyl oder C₁-C₂-Halogenalkoxy steht,
X für Fluor, Chlor, Brom, C₁-C₄-Alkyl, C₁-C₄-Alkoxy, C₃-C₄-Alkenyloxy, C₁-C₂-Halogenalkyl, C₁-C₂-Halogenalkoxy, C₂-C₄-Halogenalkenyloxy, Cyano, Nitro oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl oder Benzyloxy steht,
Y bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
Z für Wasserstoff, Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy steht,
A für C₁-C₄-Alkyl steht,
B für Wasserstoff, Methyl oder Ethyl steht,
G für Wasserstoff (a) oder für einen der Reste 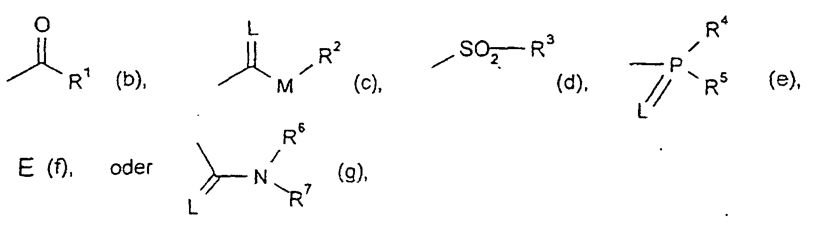 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor oder Chlor substituiertes C₁-C₆-Alkyl oder für jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl oder für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Nitro, Cyano, Fluor, Chlor, Brom, Methyl, Ethyl, n-Propyl, iso-Propyl, n-Butyl, iso-Butyl, Methoxy, Ethoxy, n-Propoxy, iso-Propoxy, Trifluormethyl, Difluormethoxy oder Trifluormethoxy steht,
X für Fluor, Chlor, Brom, Methyl, Ethyl, n-Propyl, iso-Propyl, n-Butyl, iso-Butyl, Methoxy, Ethoxy, n-Propoxy, iso-Propoxy, Trifluormethyl, Trifluormethoxy, Difluormethoxy, Cyano oder Nitro steht,
Y für Wasserstoff, Fluor, Chlor, Brom, Methyl, Ethyl, n-Propyl, iso-Propyl, n-Butyl, iso-Butyl, tert-Butyl, Methoxy, Ethoxy, n-Propoxy, iso-Propoxy, Trifluormethyl, Trifluormethoxy, Difluormethoxy, Cyano oder Nitro steht,
Z für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Difluormethoxy, Cyano oder Nitro steht,
A für Methyl oder Ethyl steht,
B für Wasserstoff oder Methyl steht,
G für Wasserstoff (a) oder für einen der Reste 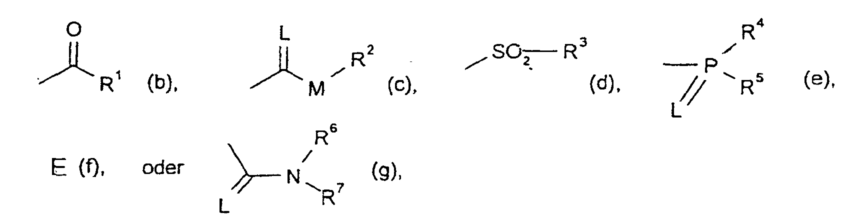 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl, oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für jeweils gegebenenfalls durch Fluor oder Chlor substituiertes Methyl, Ethyl, n-Propyl, iso-Propyl, n-Butyl, tert.-Butyl, oder für jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen und
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl, C₁-C₄-Alkoxy-C₂-C₄-alkyl oder für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, stehen, in welchem gegebenenfalls eine Methylengruppe durch Sauerstoff oder Schwefel ersetzt ist.

4. Verfahren zur Herstellung von Verbindungen der Formel (I) gemäß Anspruch 1, **dadurch gekennzeichnet, daß** man
(A) Verbindungen der Formel (I-a) 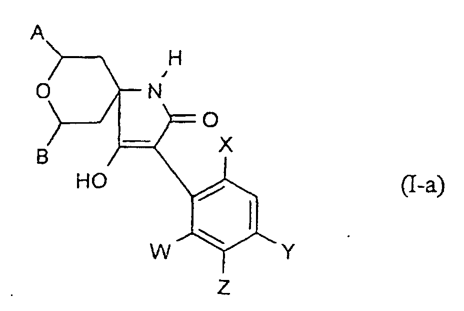 in welcher
A, B, W, X, Y und Z die in Anspruch 1 angegebenen Bedeutungen haben,
erhält, wenn man
Verbindungen der Formel (II) 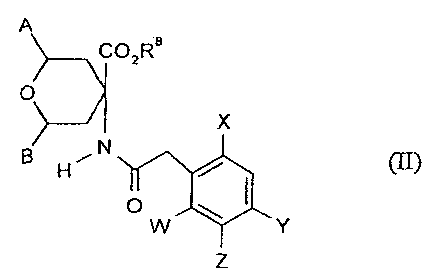 in welcher
A, B, W, X, Y und Z die in Anspruch 1 angegebenen Bedeutungen haben,
und
R⁸ für Alkyl steht,
in Gegenwart eines Verdünnungsmittels und in Gegenwart einer Base intramolekular kondensiert
und gegebenenfalls anschließend
(Bα) mit Säurehalogeniden der Formel (III) in welcher 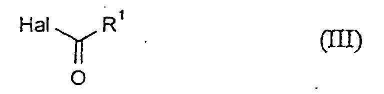
R¹ die in Anspruch 1 angegebene Bedeutung hat und
Hal für Halogen steht
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(C) mit Chlorameisensäureestern oder Chlorameisensäurethioestern der Formel (V)
R²-M-CO-Cl (V)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt;
oder
(D) mit Chlormonothioameisensäureestern oder Chlordithioameisensäureestem der Formel (VI) 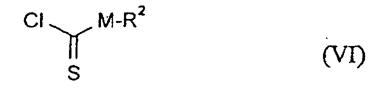 in welcher
M und R² die oben angegebenen Bedeutungen haben,
gegebenenfalls in Gegenwart eines Verdünnungsmittels und gegebenenfalls in Gegenwart eines Säurebindemittels umsetzt;
oder
(E) mit Sulfonsäurechloriden der Formel (VII)
R³-SO₂-Cl (VII)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt;
oder
(F) mit Phosphorverbindungen der Formel (VIII) 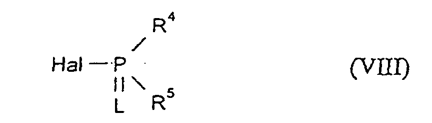 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt;
oder
(G) mit Metallverbindungen oder Aminen der Formeln (IX) oder (X)
Me(OR⁹)ₜ (IX) 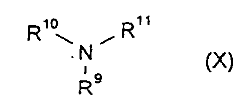
in welchen
Me für ein ein- oder zweiwertiges Metall,
t für die Zahl 1 oder 2 und
R⁹, R¹⁰, R¹¹ unabhängig voneinander für Wasserstoff oder Alkyl stehen,
gegebenenfalls in Gegenwart eines Verdünnungsmittels umsetzt;
oder
(Hα) mit Isocyanaten oder Isothiocyanaten der Formel (XI)
R⁶-N=C=L (XI)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt;
oder
β) mit Carbamidsäurechloriden oder Thiocarbamidsäurechloriden der Formel (XII) 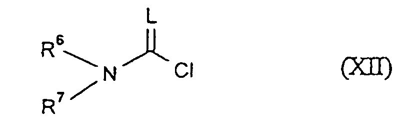 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 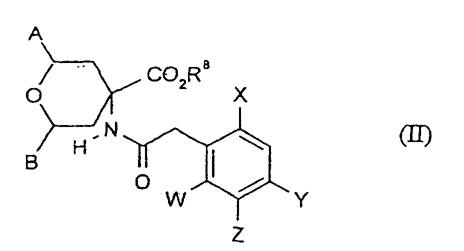 in welcher
A, B, W, X, Y und Z die in Anspruch 1 angegebenen Bedeutungen haben und
R⁸ für Alkyl steht.

6. Verbindungen der Formel (XIII) 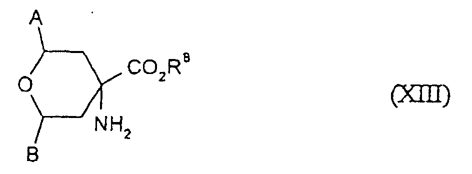 in welcher
A und B die oben angegebene Bedeutung haben und
R⁸ für Alkyl steht.

7. Verbindungen der Formel (XV) 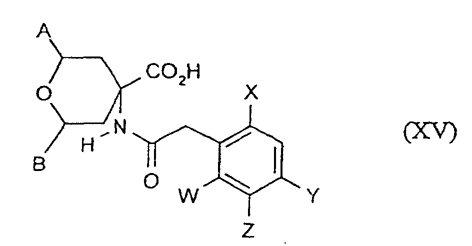 in welcher
A, B, W, X, Y und Z die in Anspruch 1 angegebenen Bedeutungen haben.

8. Verbindungen der Formel (XVI) 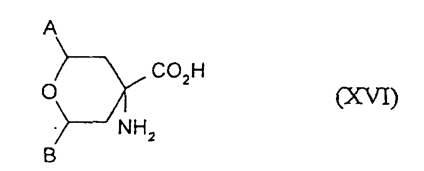 in welcher
A und B die in Anspruch 1 genannten Bedeutungen haben.

9. Verbindungen der Formel (XXII) 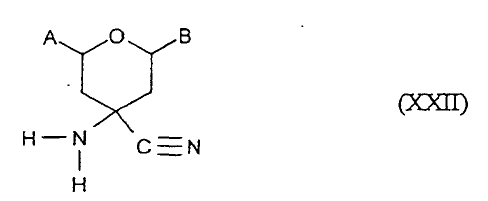 in welcher
A und B die in Anspruch 1 angegebenen Bedeutungen haben.

10. Verbindungen der Formel (XXIII) 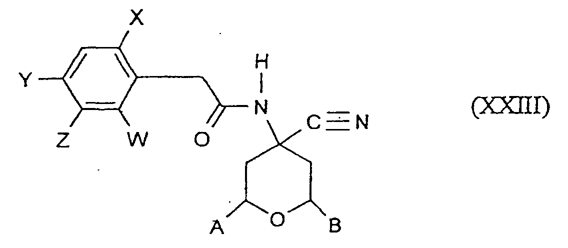 in welcher
A, B, X, Y, Z und W die in Anspruch 1 angegebenen Bedeutungen haben.

11. Schädlingsbekämpfungsmittel oder herbizide Mittel, **gekennzeichnet durch** einen Gehalt an einer Verbindung der Formel (I) gemäß Anspruch 1.

12. Verwendung von Verbindungen der Formel (I) gemäß Anspruch 1 zur Bekämpfung von Schädlingen und Unkräutern.

13. Verfahren zur Bekämpfung von Schädlingen und Unkräutern, **dadurch gekennzeichnet, daß** man Verbindungen der Formel (I) gemäß Anspruch 1 auf Schädlinge bzw. Unkräuter und/oder ihren Lebensraum einwirken läßt.

14. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 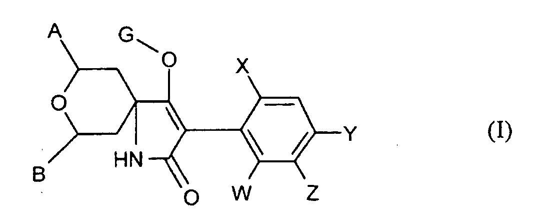 in which
W represents hydrogen, nitro, cyano, halogen, C₁-C₆-alkyl, C₂-C₆-alkenyl, C₂-C₆-alkinyl, C₁-C₆-alkoxy, C₁-C₄-haloalkyl or C₁-C₄-haloalkoxy,
X represents halogen, C₁-C₆-alkyl, C₂-C₆-alkenyl, C₂-C₆-alkinyl, C₁-C₆-alkoxy, C₃-C₆-alkenyloxy, C₁-C₄-haloalkyl, C₁-C₄-haloalkoxy, C₂-C₆-haloalkenyloxy, cyano, nitro or represents phenyl, phenoxy, phenylthio, phenyl-C₁-C₄-alkoxy or phenyl-C₁-C₄-alkylthio, each of which is optionally substituted by halogen, C₁-C₆-alkyl, C₁-C₆-alkoxy, C₁-C₄-haloalkyl, C₁-C₄-haloalkoxy, nitro or cyano,
Y represents hydrogen, halogen, C₁-C₆-alkyl, C₁-C₆-alkoxy, C₁-C₄-haloalkyl, C₁-C₄-haloalkoxy, cyano or nitro,
Z represents hydrogen, halogen, C₁-C₆-alkyl, C₁-C₆-alkoxy, C₁-C₄-haloalkyl, C₁-C₄-haloalkoxy, hydroxyl, cyano, nitro or represents phenoxy, phenylthio, thiazolyloxy, pyridinyloxy, pyrimidyloxy, pyrazolyloxy, phenyl-C₁-C₄-alkyloxy or phenyl-C₁-C₄-alkylthio, each of which is optionally substituted by halogen, C₁-C₄-alkyl, C₁-C₄-alkoxy, C₁-C₄-haloalkyl, C₁-C₄-haloalkoxy, nitro or cyano,
A represents C₁-C₆-alkyl,
B represents hydrogen or C₁-C₆-alkyl,
G represents hydrogen (a) or one of the radicals 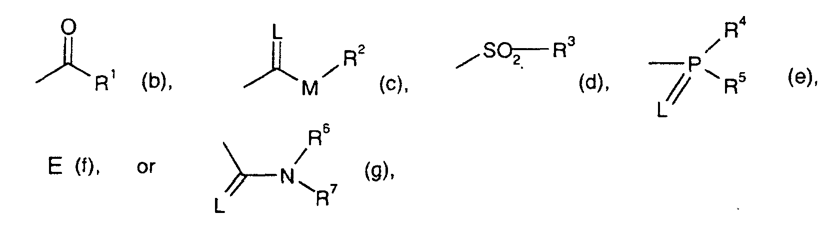 in which
E represents a metal ion equivalent or an ammonium ion,
L represents oxygen or sulphur and
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl, each of which is optionally substituted by halogen or cyano, or represents C₃-C₈-cycloalkyl which is optionally substituted by halogen, C₁-C₆-alkyl or C₁-C₆-alkoxy and in which optionally one or two not directly adjacent methylene groups are replaced by oxygen and/or sulphur,
represents phenyl which is optionally substituted by halogen, cyano, nitro, C₁-C₆-alkyl, C₁-C₆-alkoxy, C₁-C₆-haloalkyl, C₁-C₆-haloalkoxy, C₁-C₆-alkylthio or C₁-C₆-alkylsulphonyl,
represents phenyl-C₁-C₆-alkyl which is optionally substituted by halogen, nitro, cyano, C₁-C₆-alkyl, C₁-C₆-alkoxy, C₁-C₆-haloalkyl or C₁-C₆-haloalkoxy,
represents 5- or 6-membered hetaryl which is optionally substituted by halogen or C₁-C₆-alkyl and contains one or two heteroatoms from the group consisting of oxygen, sulphur and nitrogen,
represents phenoxy-C₁-C₆-alkyl which is optionally substituted by halogen or C₁-C₆-alkyl or
represents 5- or 6-membered hetaryloxy-C₁-C₆-alkyl which is optionally substituted by halogen, amino or C₁-C₆-alkyl and contains one or two heteroatoms from the group consisting of oxygen, sulphur and nitrogen,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, each of which is optionally substituted by halogen or cyano,
represents C₃-C₈-cycloalkyl which is optionally substituted by halogen, C₁-C₆-alkyl or C₁-C₆-alkoxy or
represents phenyl or benzyl, each of which is optionally substituted by halogen, cyano, nitro, C₁-C₆-alkyl, C₁-C₆-alkoxy, C₁-C₆-haloalkyl or C₁-C₆-haloalkoxy,
R³ represents C₁-C₈-alkyl which is optionally substituted by halogen or represents phenyl or benzyl, each of which is optionally substituted by halogen, C₁-C₆-alkyl, C₁-C₆-alkoxy, C₁-C₄-haloalkyl, C₁-C₄-haloalkoxy, cyano or nitro,
R⁴ and R⁵ independently of one another each represent C₁-C₈-alkyl, C₁-C₈-alkoxy, C₁-C₈-alkylamino, di-(C₁-C₈-alkyl)amino, C₁-C₈-alkylthio or C₃-C₈-alkenylthio, each of which is optionally substituted by halogen, or represent phenyl, phenoxy or phenylthio, each of which is optionally substituted by halogen, nitro, cyano, C₁-C₄-alkoxy, C₁-C₄-haloalkoxy, C₁-C₄-alkylthio, C₁-C₄-haloalkylthio, C₁-C₄-alkyl or C₁-C₄-haloalkyl,
R⁶ and R⁷ independently of one another each represent hydrogen, represent C₁-C₈-alkyl, C₃-C₈-cycloalkyl, C₁-C₈-alkoxy, C₃-C₈-alkenyl, C₁-C₈-alkoxy-C₂-C₈-alkyl, each of which is optionally substituted by halogen or cyano, or represent phenyl or benzyl, each of which is optionally subtituted by halogen, C₁-C₈-alkyl, C₁-C₈-haloalkyl or C₁-C₈-alkoxy, or together represent a C₃-C₆-alkylene radical which is optionally substituted by C₁-C₆-alkyl and in which optionally one methylene group is replaced by oxygen or sulphur.

2. Compounds of the formula (I) according to Claim 1 in which
W represents hydrogen, nitro, cyano, fluorine, chlorine, bromine, C₁-C₄-alkyl, C₁-C₄-alkoxy, C₁-C₂-haloalkyl or C₁-C₂-haloalkoxy,
X represents fluorine, chlorine, bromine, C₁-C₄-alkyl, C₁-C₄-alkoxy, C₃-C₄-alkenyloxy, C₁-C₂-haloalkyl, C₁-C₂-haloalkoxy, C₂-C₄-haloalkenyloxy, cyano, nitro or represents phenyl or benzyloxy, each of which is optionally substituted by fluorine, chlorine, bromine, C₁-C₄-alkyl, C₁-C₄-alkoxy, C₁-C₂-haloalkyl, C₁-C₂-haloalkoxy, nitro or cyano,
Y preferably represents hydrogen, fluorine, chlorine, bromine, C₁-C₄-alkyl, C₁-C₄-alkoxy, C₁-C₄-haloalkyl, C₁-C₄-haloalkoxy, cyano or nitro,
Z represents hydrogen, fluorine, chlorine, bromine, C₁-C₄-alkyl, C₁-C₄-alkoxy, C₁-C₂-haloalkyl, C₁-C₂-haloalkoxy, hydroxyl, cyano, nitro or represents phenoxy or benzyloxy, each of which is optionally substituted by fluorine, chlorine, bromine, C₁-C₄-alkyl, C₁-C₄-alkoxy, C₁-C₂-haloalkyl, C₁-C₂-haloalkoxy, nitro or cyano,
A represents C₁-C₄-alkyl,
B represents hydrogen, methyl or ethyl,
G represents hydrogen (a) or represents one of the radicals 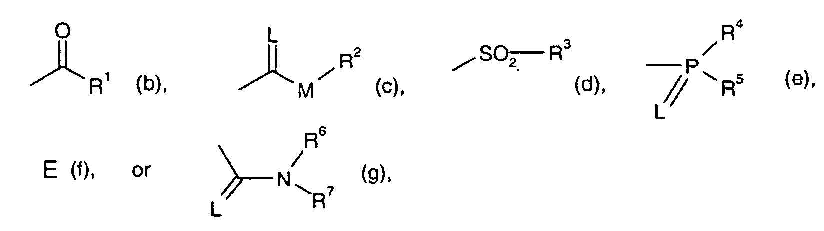 in which
E represents a metal ion equivalent or an ammonium ion,
L represents oxygen or sulphur and
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-alkyl, each of which is optionally substituted by fluorine or chlorine, or represents C₃-C₇-cycloalkyl which is optionally substituted by fluorine, chlorine, C₁-C₅-alkyl or C₁-C₅-alkoxy and in which optionally one or two not directly adjacent methylene groups are replaced by oxygen and/or sulphur,
represents phenyl which is optionally substituted by fluorine, chlorine, bromine, cyano, nitro, C₁-C₄-alkyl, C₁-C₄-alkoxy, C₁-C₃-haloalkyl, C₁-C₃-haloalkoxy, C₁-C₄-alkylthio or C₁-C₄-alkylsulphonyl,
represents phenyl-C₁-C₄-alkyl which is optionally substituted by fluorine, chlorine, bromine, C₁-C₄-alkyl, C₁-C₄-alkoxy, C₁-C₃-haloalkyl or C₁-C₃-haloalkoxy,
represents pyrazolyl, thiazolyl, pyridyl, pyrimidyl, furanyl or thienyl, each of which is optionally substituted by fluorine, chlorine, bromine or C₁-C₄-alkyl,
represents phenoxy-C₁-C₅-alkyl which is optionally substituted by fluorine, chlorine, bromine or C₁-C₄-alkyl or
represents pyridyloxy-C₁-C₅-alkyl, pyrimidyloxy-C₁-C₅-alkyl or thiazolyloxy-C₁-C₅-alkyl, each of which is optionally substituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally substituted by fluorine or chlorine,
represents C₃-C₇-cycloalkyl which is optionally substituted by fluorine, chlorine, C₁-C₄-alkyl or C₁-C₄-alkoxy or
represents phenyl or benzyl, each of which is optionally substituted by fluorine, chlorine, bromine, cyano, nitro, C₁-C₄-alkyl, C₁-C₃-alkoxy, C₁-C₃-haloalkyl or C₁-C₃-haloalkoxy,
R³ represents C₁-C₆-alkyl which is optionally substituted by fluorine or chlorine or represents phenyl or benzyl, each of which is optionally substituted by fluorine, chlorine, bromine, C₁-C₄-alkyl, C₁-C₄-alkoxy, C₁-C₂-haloalkyl, C₁-C₂-haloalkoxy, cyano or nitro,
R⁴ and R⁵ independently of one another represent C₁-C₆-alkyl, C₁-C₆-alkoxy, C₁-C₆-alkylamino, di-(C₁-C₆-alkyl)amino, C₁-C₆-alkylthio or C₃-C₄-alkenylthio, each of which is optionally substituted by fluorine or chlorine, or represent phenyl, phenoxy or phenylthio, each of which is optionally substituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or C₁-C₃-haloalkyl, and
R⁶ and R⁷ independently of one another each represent hydrogen, represent C₁-C₆-alkyl, C₃-C₆-cycloalkyl, C₁-C₆-alkoxy, C₁-C₆-alkenyl, C₁-C₆-alkoxy-C₂-C₆-alkyl, each of which is optionally substituted by fluorine or chlorine, or represent phenyl or benzyl, each of which is optionally subtituted by fluorine, chlorine, bromine, C₁-C₅-haloalkyl, C₁-C₅-alkyl or C₁-C₅-alkoxy, or together represent a C₃-C₆-alkylene radical which is optionally substituted by C₁-C₄-alkyl and in which optionally one methylene group is replaced by oxygen or sulphur.

3. Compounds of the formula (I) according to Claim 1 in which
W represents hydrogen, nitro, cyano, fluorine, chlorine, bromine, methyl, ethyl, n-propyl, isopropyl, n-butyl, isobutyl, methoxy, ethoxy, n-propoxy, isopropoxy, trifluoromethyl, difluoromethoxy or trifluoromethoxy,
X represents fluorine, chlorine, bromine, methyl, ethyl, n-propyl, isopropyl, n-butyl, isobutyl, methoxy, ethoxy, n-propoxy, isopropoxy, trifluoromethyl, trifluoromethoxy, difluoromethoxy, cyano or nitro,
Y represents hydrogen, fluorine, chlorine, bromine, methyl, ethyl, n-propyl, isopropyl, n-butyl, isobutyl, tert-butyl, methoxy, ethoxy, n-propoxy, isopropoxy, trifluoromethyl, trifluoromethoxy, difluoromethoxy, cyano or nitro,
Z represents hydrogen, fluorine, chlorine, bromine, methyl, ethyl, n-propyl, isopropyl, n-butyl, isobutyl, tert-butyl, methoxy, ethoxy, n-propoxy, isopropoxy, trifluoromethyl, trifluoromethoxy, difluoromethoxy, cyano or nitro,
A represents methyl or ethyl,
B represents hydrogen or methyl,
G represents hydrogen (a) or represents one of the radicals 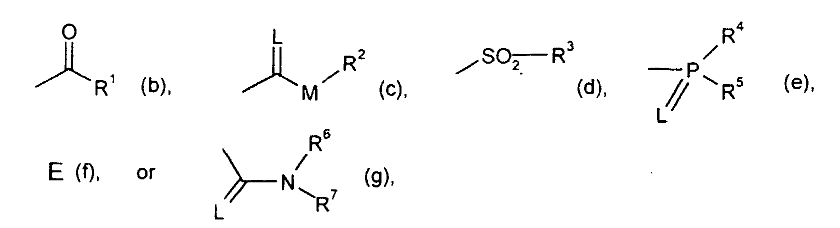 in which
E represents a metal ion equivalent or an ammonium ion,
L represents oxygen or sulphur and
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl, poly-C₁-C₄-alkoxy-C₁-C₄-alkyl, each of which is optionally substituted by fluorine or chlorine, or represents C₃-C₆-cycloalkyl which is optionally substituted by fluorine, chlorine, methyl, ethyl, n-propyl, isopropyl, n-butyl, isobutyl, tert-butyl, methoxy, ethoxy, n-propoxy or isopropoxy and in which optionally one or two not directly adjacent methylene groups are replaced by oxygen and/or sulphur,
represents phenyl which is optionally substituted by fluorine, chlorine, bromine, cyano, nitro, methyl, ethyl, n-propyl, i-propyl, methoxy, ethoxy, trifluoromethyl, trifluoromethoxy, methylthio, ethylthio, methylsulphonyl or ethylsulphonyl,
represents benzyl which is optionally substituted by fluorine, chlorine, bromine, methyl, ethyl, n-propyl, i-propyl, methoxy, ethoxy, trifluoromethyl or trifluoromethoxy,
represents furanyl, thienyl or pyridyl, each of which is optionally substituted by fluorine, chlorine, bromine, methyl or ethyl,
represents phenoxy-C₁-C₄-alkyl which is optionally substituted by fluorine, chlorine, methyl or ethyl or
represents pyridyloxy-C₁-C₄-alkyl, pyrimidyloxy-C₁-C₄-alkyl or thiazolyloxy-C₁-C₄-alkyl, each of which is optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, each of which is optionally substituted by fluorine or chlorine,
represents C₃-C₆-cycloalkyl which is optionally substituted by fluorine, chlorine, methyl, ethyl, n-propyl, isopropyl or methoxy,
or represents phenyl or benzyl, each of which is optionally substituted by fluorine, chlorine, cyano, nitro, methyl, ethyl, n-propyl, i-propyl, methoxy, ethoxy, trifluoromethyl or trifluoromethoxy,
R³ represents methyl, ethyl, n-propyl, isopropyl, n-butyl, tert-butyl, each of which is optionally substituted by fluorine or chlorine, or represents phenyl or benzyl, each of which is optionally substituted by fluorine, chlorine, bromine, methyl, ethyl, isopropyl, tert-butyl, methoxy, ethoxy, isopropoxy, trifluoromethyl, trifluoromethoxy, cyano or nitro,
R⁴ and R⁵ independently of one another represent C₁-C₄-alkyl, C₁-C₄-alkoxy, C₁-C₄-alkylamino, di-(C₁-C₄-alkyl)amino or C₁-C₄-alkylthio, each of which is optionally substituted by fluorine or chlorine, or represent phenyl, phenoxy or phenylthio, each of which is optionally substituted by fluorine, chlorine, bromine, nitro, cyano, methyl, methoxy, trifluoromethyl or trifluoromethoxy and
R⁶ and R⁷ independently of one another represent hydrogen, represent C₁-C₄-alkyl, C₃-C₆-cycloalkyl, C₁-C₄-alkoxy, C₃-C₄-alkenyl, C₁-C₄-alkoxy-C₂-C₄-alkyl, each of which is optionally substituted by fluorine or chlorine, or represent phenyl or benzyl, each of which is optionally subtituted by fluorine, chlorine, bromine, methyl, methoxy or trifluoromethyl, or together represent a C₅-C₆-alkylene radical which is optionally substituted by methyl or ethyl and in which optionally one methylene group is replaced by oxygen or sulphur.

4. Process for preparing compounds of the formula (I) according to Claim 1, **characterized in that**
(A) compounds of the formula (I-a) 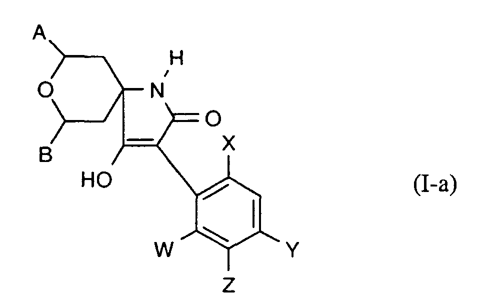 in which
A, B, W, X, Y and Z are each as defined in Claim 1,
are obtained when
compounds of the formula (II) 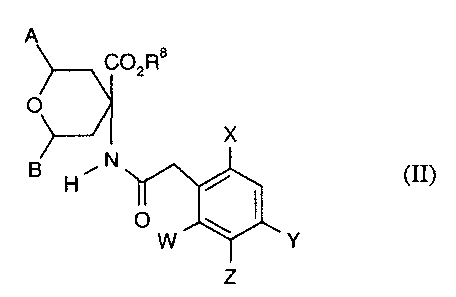 in which
A, B, W, X, Y and Z are each as defined in Claim 1
and
R⁸ represents alkyl,
are intramolecularly condensed in the presence of a diluent and in the presence of a base
and, if appropriate, subsequently
(Bα) reacted with acyl halides of the formula (III) 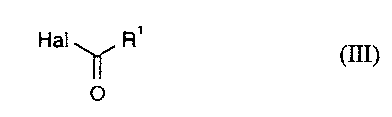 in which
R¹ is as defined in Claim 1 and
Hal represents halogen
or
β) reacted with carboxylic anhydrides of the formula (IV)
R¹-CO-O-CO-R¹ (IV)
in which
R¹ is as defined above,
if appropriate in the presence of a diluent and if appropriate in the presence of an acid binder;
or
(C) reacted with chloroformic esters or chloroformic thioesters of the formula (V)
R²-M-CO-Cl (V)
in which
R² and M are each as defined in Claim 1,
if appropriate in the presence of a diluent and if appropriate in the presence of an acid binder;
or
(D) reacted with chloromonothioformic esters or chlorodithioformic esters of the formula (VI) 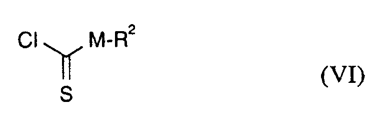 in which
M and R² are each as defined above,
if appropriate in the presence of a diluent and if appropriate in the presence of an acid binder;
or
(E) reacted with sulphonyl chlorides of the formula (VII)
R³-SO₂-Cl (VII)
in which
R³ is as defined in Claim 1,
if appropriate in the presence of a diluent and if appropriate in the presence of an acid binder;
or
(F) reacted with phosphorus compounds of the formula (VIII) 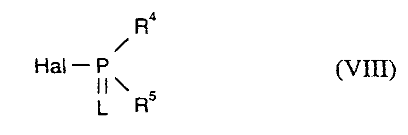 in which
L, R⁴ and R⁵ are each as defined in Claim 1 and
Hal represents halogen,
if appropriate in the presence of a diluent and if appropriate in the presence of an acid binder;
or
(G) reacted with metal compounds or amines of the formulae (IX) or (X)
Me(OR⁹)ₜ (IX) 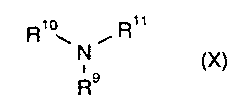
in which
Me represents a mono- or divalent metal,
t represents the number 1 or 2 and
R9, R¹⁰, R¹¹ independently of one another each represent hydrogen or alkyl,
if appropriate in the presence of a diluent;
or
(Hα) reacted with isocyanates or isothiocyanates of the formula (XI)
R⁶-N=C=L (XI)
in which
R⁶ and L are each as defined in Claim 1,
if appropriate in the presence of a diluent and if appropriate in the presence of a catalyst;
or
β) reacted with carbamoyl chlorides or thiocarbamoyl chlorides of the formula (XII) 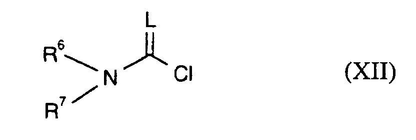 in which
L, R⁶ and R⁷ are each as defined in Claim 1,
if appropriate in the presence of a diluent and if appropriate in the presence of an acid binder.

5. Compounds of the formula (II) 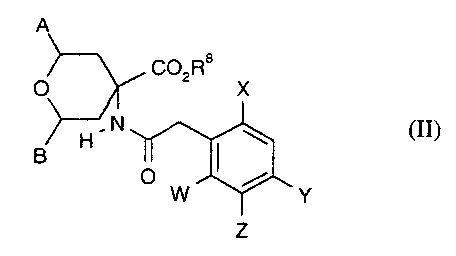 in which
A, B, W, X, Y and Z are each as defined in Claim 1 and
R⁸ represents alkyl.

6. Compounds of the formula (XIII) 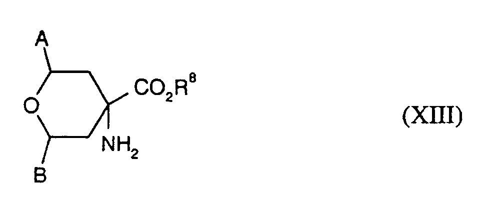 in which
A and B are each as defined above and
R⁸ represents alkyl.

7. Compounds of the formula (XV) 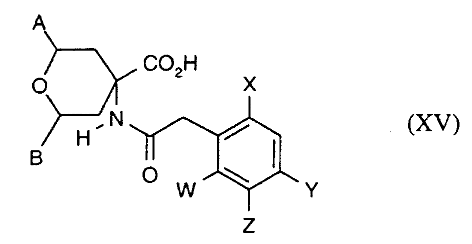 in which
A, B, W, X, Y and Z are each as defined in Claim 1.

8. Compounds of the formula (XVI) in which 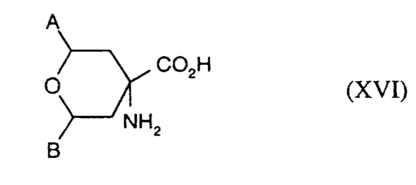
A and B are each as defined in Claim 1.

9. Compounds of the formula (XXII) in which 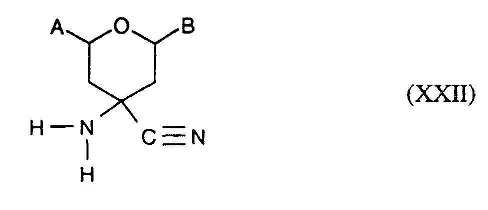
A and B are each as defined in Claim 1.

10. Compounds of the formula (XXIII) 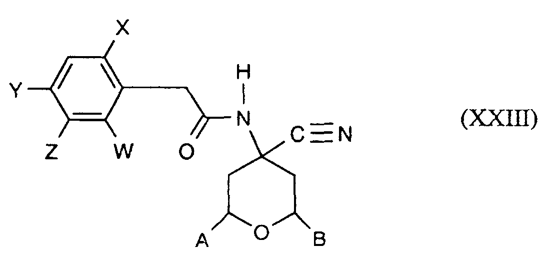 in which
A, B, X, Y, Z and W are each as defined in Claim 1.

11. Pesticides or herbicides, **characterized in that** they contain a compound of the formula (I) according to Claim 1.

12. The use of compounds of the formula (I) according to Claim 1 for controlling pests and weeds.

13. Method for controlling pests and weeds, **characterized in that** compounds of the formula (I) according to Claim 1 are allowed to act on pests or weeds and/or their habitat.

14. Process for preparing pesticides and herbicides, **characterized in that** compounds of the formula (I) according to Claim 1 are mixed with extenders and/or surfactants.

## Revendications

1. Composés de la formule (I) : 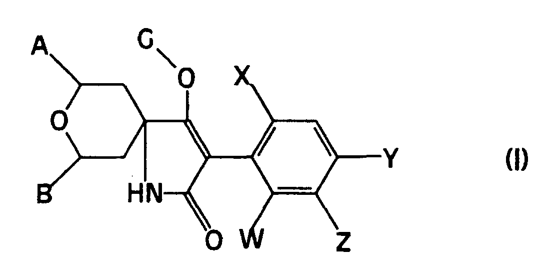 dans laquelle :
W représente l'atome d'hydrogène, le radical nitro, cyano, un atome d'halogène, un radical alcoyle en C₁-C₆, alcényle en C₂-C₆, alcynyle en C₂-C₆, alcoxy en C₁-C₆, halogénoalcoyle en C₁-C₄ ou halogénoalcoxy en C₁-C₄,
X représente un atome d'halogène, un radical alcoyle en C₁-C₆, alcényle en C₂-C₆, alcynyle en C₂-C₆, alcoxy en C₁-C₆, alcényloxy en C₃-C₆, halogénoalcoyle en C₁-C₄, halogénoalcoxy en C₁-C₄, halogénoalcényloxy en C₂-C₆, le radical cyano, nitro ou représente un radical phényle, phénoxy, phénylthio, phényl(alcoxy en C₁-C₄) ou phényl(alcoylthio en C₁-C₄), chaque fois le cas échéant substitué par un atome d'halogène, un radical alcoyle en C₁-C₆, alcoxy en C₁-C₆, halogénoalcoyle en C₁-C₄, halogénoalcoxy en C₁-C₄, le radical nitro ou cyano,
Y représente l'atome d'hydrogène, un atome d'halogène, un radical alcoyle en C₁-C₆, alcoxy en C₁-C₆, halogénoalcoyle en C₁-C₄, halogénoalcoxy en C₁-C₄, le radical cyano ou nitro,
Z représente l'atome d'hydrogène, un atome d'halogène, un radical alcoyle en C₁-C₆, alcoxy en C₁-C₆, halogénoalcoyle en C₁-C₄, halogénoalcoxy en C₁-C₄, le radical hydroxyle, cyano, nitro ou représente un radical phénoxy, phénylthio, thiazolyloxy, pyridinyloxy, pyrimidyloxy, pyrazolyloxy, phényl(alcoxy en C₁-C₄) ou phényl(alcoylthio en C₁-C₄), chaque fois le cas échéant substitué par un atome d'halogène, un radical alcoyle en C₁-C₄, alcoxy en C₁-C₄, halogénoalcoyle en C₁-C₄, halogénoalcoxy en C₁-C₄, le radical nitro ou cyano,
A représente un radical alcoyle en C₁-C₆,
B représente l'atome d'hydrogène ou un radical alcoyle en C₁-C₆,
G représente l'atome d'hydrogène (a) ou un des restes 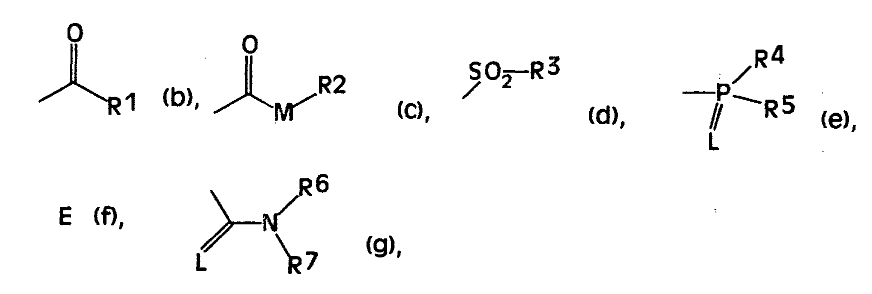 dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₂₀, alcényle en C₂-C₂₀, (alcoxy en C₁-C₈)alcoyle en C₁-C₈, (alcoythio en C₁-C₈)alcoyle en C₁-C₈ ou poly(alcoxy en C₁-C₈)alcoyle en C₁-C₈, chaque fois le cas échéant substitué par un atome d'halogène ou le radical cyano, ou représente un radical cycloalcoyle en C₃-C₈, le cas échéant substitué par un atome d'halogène, un radical alcoyle en C₁-C₆ ou alcoxy en C₁-C₆, et/ dans lequel le cas échéant, un ou deux radicaux méthylène non directement voisins sont remplacés par l'atome d'oxygène et/ou de soufre,
représente le radical phényle, le cas échéant substitué par un atome d'halogène, le radical cyano, nitro, un radical alcoyle en C₁-C₆, alcoxy en C₁-C₆, halogénoalcoyle en C₁-C₆, halogénoalcoxy en C₁-C₆, alcoylthio en C₁-C₆ ou alcoylsulfonyle en C₁-C₆,
représente le radical phényl(alcoyle en C₁-C₆), le cas échéant substitué par un atome d'halogène, le radical nitro, cyano, un radical alcoyle en C₁-C₆, alcoxy en C₁-C₆, halogénoalcoyle en C₁-C₆ ou halogénoalcoxy en C₁-C₆,
représente un radical hétéroaryle à 5 ou 6 membres, avec un ou deux hétéroatomes de la série des atomes d'oxygène, de soufre et d'azote, le cas échéant substitué par un atome d'halogène ou un radical alcoyle en C₁-C₆,
représente le radical phénoxy(alcoyle en C₁-C₆), le cas échéant substitué par un atome d'halogène ou un radical alcoyle en C₁-C₆,
un radical hétéroaryloxy(alcoyle en C₁-C₆) à 5 ou 6 membres, avec un ou deux hétéroatomes de la série des atomes d'oxygène, de soufre et d'azote, le cas échéant substitué par un atome d'halogène, le radical amino ou un radical alcoyle en C₁-C₆,
R² représente un radical alcoyle en C₁-C₂₀, alcényle en C₂-C₂₀, (alcoxy en C₁-C₈)alcoyle en C₂-C₈ ou poly(alcoxy en C₁-C₈)alcoyle en C₂-C₈, chaque fois le cas échéant substitué par un atome d'halogène ou le radical cyano,
représente un radical cycloalcoyle en C₃-C₈, le cas échéant substitué par un atome d'halogène, un radical alcoyle en C₁-C₆ ou alcoxy en C₁-C₆, ou
représente le radical phényle ou benzyle, chaque fois le cas échéant substitué par un atome d'halogène, le radical cyano, nitro, un radical alcoyle en C₁-C₆, alcoxy en C₁-C₆, halogénoalcoyle en C₁-C₆ ou halogénoalcoxy en C₁-C₆,
R³ représente un radical alcoyle en C₁-C₆ le cas échéant substitué par un atome d'halogène, ou représente le radical phényle ou benzyle, chaque fois le cas échéant substitué par un atome d'halogène, un radical alcoyle en C₁-C₆, alcoxy en C₁-C₆, halogénoalcoyle en C₁-C₄, halogénoalcoxy en C₁-C₄ ou le radical cyano ou nitro,
R⁴ et R⁵ représentent indépendamment l'un de l'autre, un radical alcoyle en C₁-C₈, alcoxy en C₁-C₈, alcoylamino en C₁-C₈, di (alcoyl en C₁-C₈)amino, alcoylthio en C₁-C₈ ou alcénylthio en C₃-C₈, le cas échéant substitué par un atome d'halogène, ou représente un radical phényle, phénoxy ou phénylthio, chaque fois le cas échéant substitué par un atome d'halogène, le radical nitro ou cyano, un radical alcoxy en C₁-C₄, halogénoalcoxy en C₁-C₄, alcoylthio en C₁-C₄, halogénoalcoylthio en C₁-C₄, alcoyle en C₁-C₄ ou halogénoalcoyle en C₁-C₄,
R⁶ et R⁷ représentent indépendamment l'un de l'autre, l'atome d'hydrogène, un radical alcoyle en C₁-C₆, cycloalcoyle en C₃-C₈, alcoxy en C₁-C₈, alcényle en C₃-C₈, (alcoxy en C₁-C₈)alcoyle en C₂-C₈, chaque fois le cas échéant substitué par un atome d'halogène ou le radical cyano, ou représentent le radical phényle ou benzyle, chaque fois le cas échéant substitué par un atome d'halogène, un radical alcoyle en C₁-C₈, halogénoalcoyle en C₁-C₈ ou alcoxy en C₁-C₈, ou ensemble, représentent un reste alcoylène en C₃-C₆, dans lequel un radical méthylène est le cas échéant, remplacé par l'atome d'oxygène ou de soufre, substitué le cas échéant par un radical alcoyle en C₁-C₆.

2. Composés de la formule (I) suivant la revendication 1, dans lesquels :
W représente l'atome d'hydrogène, le radical nitro, cyano, l'atome de fluor, de chlore, de brome, un radical alcoyle en C₁-C₄, alcoxy en C₁-C₄, halogénoalcoyle en C₁-C₂ ou halogénoalcoxy en C₁-C₂,
X représente l'atome de fluor, de chlore, de brome, un radical alcoyle en C₁-C₄, alcoxy en C₁-C₄, alcényloxy en C₃-C₄, halogénoalcoyle en C₁-C₂, halogénoalcoxy en C₁-C₂, halogénoalcényloxy en C₂-C₄, le radical cyano, nitro ou représente le radical phényle, ou benzyloxy, chaque fois le cas échéant substitué par l'atome de fluor, de chlore, de brome, un radical alcoyle en C₁-C₄, alcoxy en C₁-C₄, halogénoalcoyle en C₁-C₂, halogénoalcoxy en C₁-C₂, le radical nitro ou cyano,
Y représente de préférence, l'atome d'hydrogène, l'atome de fluor, de chlore, de brome, un radical alcoyle en C₁-C₄, alcoxy en C₁-C₄, halogénoalcoyle en C₁-C₂, halogénoalcoxy en C₁-C₂, le radical cyano ou nitro,
Z représente l'atome d'hydrogène, l'atome de fluor, de chlore, de brome, un radical alcoyle en C₁-C₄, alcoxy en C₁-C₄, halogénoalcoyle en C₁-C₂, halogénoalcoxy en C₁-C₂, le radical hydroxyle, cyano, nitro ou représente le radical phénoxy ou benzyloxy, chaque fois le cas échéant substitué par l'atome de fluor, de chlore, de brome, un radical alcoyle en C₁-C₄, alcoxy en C₁-C₄, halogénoalcoyle en C₁-C₂, halogénoalcoxy en C₁-C₂, le radical nitro ou cyano,
A représente un radical alcoyle en C₁-C₄,
B représente l'atome d'hydrogène, le radical méthyle ou éthyle,
G représente l'atome d'hydrogène (a) ou un des restes 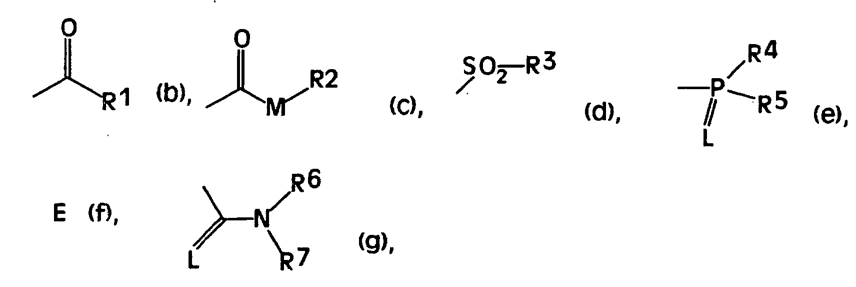 dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₁₆, alcényle en C₂-C₁₆, (alcoxy en C₁-C₆)alcoyle en C₁-C₆, (alcoythio en C₁-C₆)alcoyle en C₁-C₆ ou poly (alcoxy en C₁-C₆)alcoyle en C₁-C₆, chaque fois le cas échéant substitué par l'atome de fluor ou de chlore, ou représente un radical cycloalcoyle en C₃-C₇, le cas échéant substitué par l'atome de fluor ou de chlore, un radical alcoyle en C₁-C₅ ou alcoxy en C₁-C₅, et/dans lequel le cas échéant, un ou deux radicaux méthylène non directement voisins sont remplacés par l'atome d'oxygène et/ou de soufre,
représente le radical phényle, le cas échéant substitué par l'atome de fluor, de chlore, de brome, le radical cyano, nitro, un radical alcoyle en C₁-C₄, alcoxy en C₁-C₄, halogénoalcoyle en C₁-C₃, halogénoalcoxy en C₁-C₃, alcoylthio en C₁-C₄ ou alcoylsulfonyle en C₁-C₄,
représente un radical phényl(alcoyle en C₁-C₄), le cas échéant substitué par l'atome de fluor, de chlore, de brome, un radical alcoyle en C₁-C₄, alcoxy en C₁-C₄, halogénoalcoyle en C₁-C₃ ou halogénoalcoxy en C₁-C₃,
représente le radical pyrazolyle, thiazolyle, pyridyle, pyrimidyle, furannyle ou thiényle chaque fois le cas échéant substitué par l'atome de fluor, de chlore, de brome ou un radical alcoyle en C₁-C₄,
représente le radical phénoxy(alcoyle en C₁-C₅), le cas échéant substitué par l'atome de fluor, de chlore, de brome ou un radical alcoyle en C₁-C₄,
un radical pyridyloxy(alcoyle en C₁-C₅), pyrimidyloxy(alcoyle en C₁-C₅) ou thiazolyloxy(alcoyle en C₁-C₅), chaque fois le cas échéant substitué par l'atome de fluor, de chlore, de brome, le radical amino ou un radical alcoyle en C₁-C₄,
R² représente un radical alcoyle en C₁-C₁₆, alcényle en C₂-C₁₆, (alcoxy en C₁-C₆)alcoyle en C₂-C₆ ou poly(alcoxy en C₁-C₆)alcoyle en C₂-C₆, chaque fois le cas échéant substitué par l'atome de fluor ou de chlore,
représente un radical cycloalcoyle en C₃-C₇, le cas échéant substitué par l'atome de fluor ou de chlore, un radical alcoyle en C₁-C₄ ou alcoxy en C₁-C₄, ou
représente le radical phényle ou benzyle, chaque fois le cas échéant substitué par l'atome de fluor, de chlore, de brome, le radical cyano, nitro, un radical alcoyle en C₁-C₄, alcoxy en C₁-C₃, halogénoalcoyle en C₁-C₃ ou halogénoalcoxy en C₁-C₃,
R³ représente un radical alcoyle en C₁-C₆ le cas échéant substitué par l'atome de fluor ou de chlore, ou représente le radical phényle ou benzyle, chaque fois le cas échéant substitué par l'atome de fluor, de chlore, de brome, un radical alcoyle en C₁-C₄, alcoxy en C₁-C₄, halogénoalcoyle en C₁-C₂, halogénoalcoxy en C₁-C₂ ou le radical cyano ou nitro,
R⁴ et R⁵ représentent indépendamment l'un de l'autre, un radical alcoyle en C₁-C₆, alcoxy en C₁-C₆, alcoylamino en C₁-C₆, di (alcoyl en C₁-C₆) amino, alcoylthio en C₁-C₆ ou alcénylthio en C₃-C₄, le cas échéant substitué par l'atome de fluor ou de chlore, ou représente un radical phényle, phénoxy ou phénylthio, chaque fois le cas échéant substitué par l'atome de fluor, de chlore, de brome, le radical nitro ou cyano, un radical alcoxy en C₁-C₃, halogénoalcoxy en C₁-C₃, alcoylthio en C₁-C₃, halogénoalcoylthio en C₁-C₃, alcoyle en C₁-C₃ ou halogénoalcoyle en C₁-C₃,
R⁶ et R⁷ représentent indépendamment l'un de l'autre, l'atome d'hydrogène, un radical alcoyle en C₁-C₆, cycloalcoyle en C₃-C₆, alcoxy en C₁-C₆, alcényle en C₃-C₆, (alcoxy en C₁-C₆)alcoyle en C₂-C₆, chaque fois le cas échéant substitué par l'atome de fluor ou de chlore, ou représentent le radical phényle ou benzyle, chaque fois le cas échéant substitué par l'atome de fluor, de chlore, de brome, un radical halogénoalcoyle en C₁-C₅, alcoyle en C₁-C₅ ou alcoxy en C₁-C₅, ou ensemble, représentent un reste alcoylène en C₃-C₆, dans lequel un radical méthylène est le cas échéant, remplacé par l'atome d'oxygène ou de soufre, substitué la cas échéant par un radical alcoyle en C₁-C₄.

3. Composés de la formule (I) suivant la revendication 1, dans lesquels :
W représente l'atome d'hydrogène, le radical nitro, cyano, l'atome de fluor, de chlore, de brome, le radical méthyle, éthyle, n-propyle, isopropyle, n-butyle, isobutyle, méthoxy, éthoxy, n-propoxy, isopropoxy, trifluorométhyle, difluorométhyle ou triflorométhoxy,
X représente l'atome de fluor, de chlore, de brome, le radical méthyle, éthyle, n-propyle, isopropyle, n-butyle, isobutyle, méthoxy, éthoxy, n-propoxy, isopropoxy, trifluorométhyle, triflorométhoxy, difluorométhoxy, le radical nitro ou cyano,
Y représente l'atome d'hydrogène, l'atome de fluor, de chlore, de brome, le radical méthyle, éthyle, n-propyle, isopropyle, n-butyle, isobutyle, t-butyle, méthoxy, éthoxy, n-propoxy, isopropoxy, trifluorométhyle, triflorométhoxy, difluorométhoxy, le radical cyano ou nitro,
Z représente l'atome d'hydrogène, l'atome de fluor, de chlore, de brome, le radical méthyle, éthyle, n-propyle, isopropyle, n-butyle, isobutyle, t-butyle, méthoxy, éthoxy, n-propoxy, isopropoxy, trifluorométhyle, triflorométhoxy, difluorométhoxy, le radical nitro ou cyano,
A représente le radical méthyle ou éthyle,
B représente l'atome d'hydrogène ou le radical méthyle,
G représente l'atome d'hydrogène (a) ou un des restes 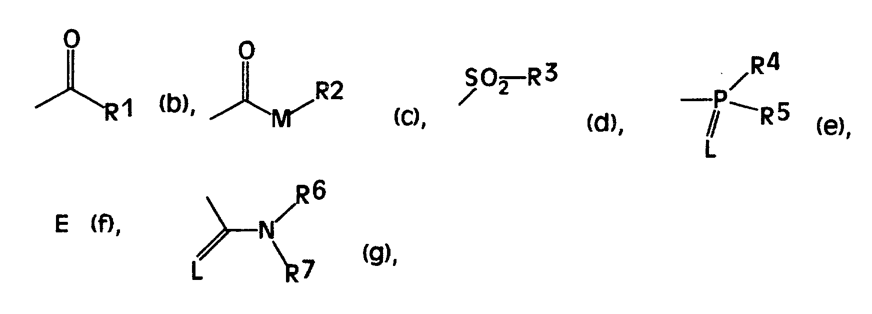 dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₁₄, alcényle en C₂-C₁₄, (alcoxy en C₁-C₄)alcoyle en C₁-C₆, (alcoythio en C₁-C₄)alcoyle en C₁-C₆ ou poly(alcoxy en C₁-C₄)alcoyle en C₁-C₄, chaque fois le cas échéant substitué par l'atome de fluor ou de chlore, ou représente un radical cycloalcoyle en C₃-C₃, le cas échéant substitué par l'atome de fluor ou de chlore, le radical méthyle, éthyle, n-propyle, isopropyle, n-butyle, isobutyle, t-butyle, méthoxy, éthoxy, n-propoxy ou isopropoxy, et/dans lequel le cas échéant, un ou deux radicaux méthylène non directement voisins sont remplacés par l'atome d'oxygène et/ou de soufre,
représente le radical phényle, le cas échéant substitué par l'atome de fluor, de chlore, de brome, le radical cyano, nitro, méthyle, éthyle, n-propyle, isopropyle, méthoxy, éthoxy, trifluorométhyle, triflorométhoxy, méthylthio, éthylthio, méthylsulfonyle
ou éthylsulfonyle,
représente le radical benzyle, le cas échéant substitué par l'atome de fluor, de chlore, de brome, le radical méthyle, éthyle, n-propyle, isopropyle, méthoxy, éthoxy, trifluorométhyle ou triflorométhoxy,
représente le radical furannyle, thiényle ou pyridyle, chaque fois le cas échéant substitué par l'atome de fluor, de chlore, de brome, le radical méthyle ou éthyle,
représente le radical phénoxy(alcoyle en C₁-C₄), le cas échéant substitué par l'atome de fluor ou de chlore, le radical méthyle ou éthyle, ou
un radical pyridyloxy(alcoyle en C₁-C₄), pyrimidyloxy(alcoyle en C₁-C₄) ou thiazolyloxy(alcoyle en C₁-C₄), chaque fois le cas échéant substitué par l'atome de fluor ou de chlore, le radical amino, méthyle ou éthyle,
R² représente un radical alcoyle en C₁-C₁₄, alcényle en C₂-C₁₄, (alcoxy en C₁-C₄)alcoyle en C₂-C₆ ou poly(alcoxy en C₁-C₄)alcoyle en C₂-C₆, chaque fois le cas échéant substitué par l'atome de fluor ou de chlore,
représente un radical cycloalcoyle en C₃-C₆, le cas échéant substitué par l'atome de fluor, de chlore, de brome, le radical méthyle, éthyle, n-propyle, isopropyle ou méthoxy, ou
représente le radical phényle ou benzyle, chaque fois le cas échéant substitué par l'atome de fluor, de chlore, le radical cyano, nitro, le radical méthyle, éthyle, n-propyle, isopropyle, méthoxy, éthoxy, trifluorométhyle, ou triflorométhoxy,
R³ représente le radical méthyle, éthyle, n-propyle, isopropyle, n-butyle, t-butyle, chaque fois le cas échéant substitué par l'atome de fluor ou de chlore, ou représente le radical phényle ou benzyle, chaque fois le cas échéant substitué par l'atome de fluor, de chlore, de brome, le radical méthyle, éthyle, isopropyle, t-butyle, méthoxy, éthoxy, isopropoxy, trifluorométhyle, triflorométhoxy, ou le radical cyano ou nitro,
R⁴ et R⁵ représentent indépendamment l'un de l'autre, un radical alcoyle en C₁-C₄, alcoxy en C₁-C₄, alcoylamino en C₁-C₄, di (alcoyl en C₁-C₄)amino ou alcoylthio en C₁-C₄, chaque fois le cas échéant substitué par l'atome de fluor ou de chlore, ou représente le radical phényle, phénoxy ou phénylthio, chaque fois le cas échéant substitué par l'atome de fluor, de chlore, de brome, le radical nitro, cyano, méthyle, méthoxy, trifluorométhyle ou trifluorométhoxy, et
R⁶ et R⁷ représentent indépendamment l'un de l'autre, l'atome d'hydrogène, un radical alcoyle en C₁-C₄, cycloalcoyle en C₃-C₆, alcoxy en C₁-C₄, alcenyle en C₃-C₄, (alcoxy en C₁-C₄)alcoyle en C₂-C₈, chaque fois le cas échéant substitué par l'atome de fluor ou de chlore, ou représentent le radical phényle ou benzyle, chaque fois le cas échéant substitué par l'atome de fluor, de chlore, de brome, le radical méthyle, méthoxy ou trifluorométhyle, ou ensemble, représentent un reste alcoylène en C₅-C₆, dans lequel un radical méthylène est le cas échéant, remplacé par l'atome d'oxygène ou de soufre, substitué la cas échéant par le radical méthyle ou éthyle.

4. Procédé de préparation des composés de la formule (I) suivant la revendication 1, **caractérisé en ce que** :
(A) on obtient des composés de la formule (I-a) : 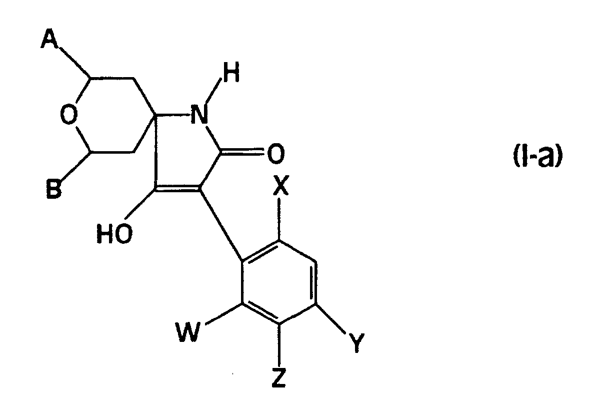 dans laquelle :
A, B, W, X, Y et Z ont les significations indiquées à la revendication 1,
lorsque l'on condense de manière intramoléculaire, des composés de la formule (II) : 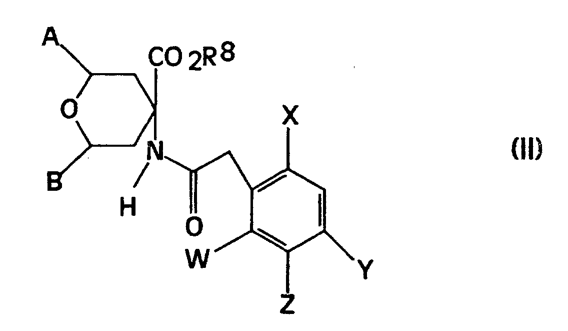
dans laquelle :
A, B, W, X, Y et Z ont les significations indiquées à la revendication 1,
et
R⁸ représente un radical alcoyle,
en présence d'un agent de dilution et en présence d'une base,
et ensuite, le cas échéant
(Bα) on fait réagir avec des halogénures d'acide de la formule (III) : 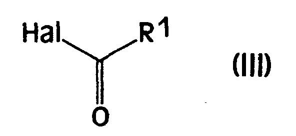 dans laquelle :
R¹ a la signification donnée à la revendication 1, et
Hal représente un atome d'halogène, ou
β) avec des anhydrides d'acide carboxylique de la formule (IV) :
R¹ - CO - O - CO- - R¹ (IV)
dans laquelle
R¹ a la signification donnée ci-dessus,
le cas échéant en présence d'un agent de dilution et le cas échéant, en présence d'un agent liant les acides ;
ou
(C) on fait réagir avec des esters de l'acide chloroformique ou des thioesters de l'acide chloroformique de la formule (V) :
R² - M - CO - Cl (V)
dans laquelle:
R² et M ont les significations données à la revendication 1, et
le cas échéant en présence d'un agent de dilution et le cas échéant, en présence d'un agent liant les acides ;
ou
(D) on fait réagir avec des esters de l'acide chloromonothioformique ou des esters de l'acide chlorodithioformique de la formule (VI) : 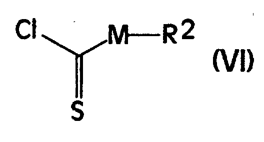 dans laquelle:
R² et M ont les significations données ci-dessus, et
le cas échéant en présence d'un agent de dilution et le cas échéant, en présence d'un agent liant les acides ;
ou
(E) on fait réagir avec des chlorures d'acide sulfonique de la formule (VII) :
R³ - SO₂ - Cl (VII)
dans laquelle:
R³ a la signification donnée à la revendication 1, et
le cas échéant en présence d'un agent de dilution et le cas échéant, en présence d'un agent liant les acides ;
ou
(F) on fait réagir avec des composés du phosphore de la formule (VIII) : 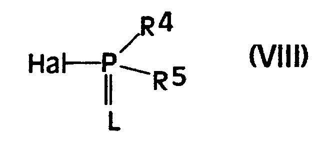 dans laquelle:
L, R⁴ et R⁵ ont les significations données à la revendication 1, et
Hal représente un atome d'halogène,
le cas échéant en présence d'un agent de dilution et le cas échéant, en présence d'un agent liant les acides ;
ou
(G) on fait réagir avec des composés métalliques ou des amines des formules (IX) ou (X) :
Me(OR⁹)ₜ (IX) 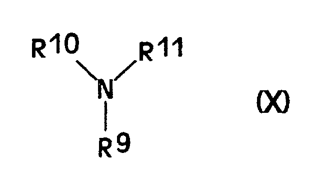
dans laquelle:
Me représente un métal mono- ou bivalent,
t représente le nombre 1 ou 2, et
R⁹, R¹⁰ et R¹¹ représentent indépendamment l'un de l'autre, l'atome d'hydrogène ou un radical alcoyle,
le cas échéant en présence d'un agent de dilution ;
ou
(Hα) on fait réagir des isocyanates ou des isthiocyanates de la formule (XI) :
R⁶ - N = C = L (XI)
dans laquelle :
R⁶ et L ont les significations données à la revendication 1,
le cas échéant en présence d'un agent de dilution et le cas échéant, en présence d'un catalyseur ;
ou
β) avec des chlorures d'acide carbamique ou des chlorures d'acide thiocarbamique de la formule (XII) : 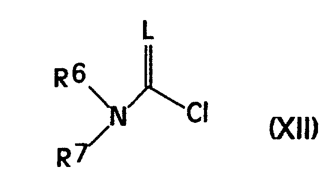 dans laquelle :
L, R⁶ et R⁷ ont les significations données à la revendication 1,
le cas échéant en présence d'un agent de dilution et le cas échéant, en présence d'un agent liant les acides.

5. Composés de la formule (II) : 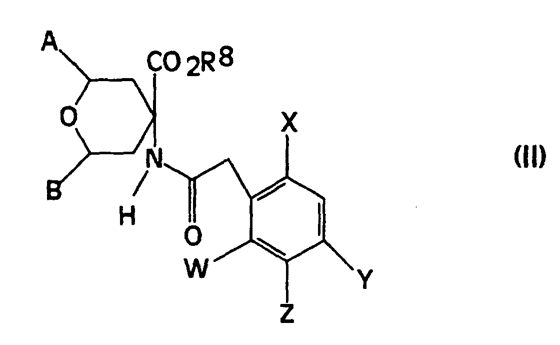 dans laquelle :
A, B, W, X, Y et Z ont les significations indiquées à la revendication 1,
et
R⁸ représente un radical alcoyle.

6. Composés de la formule (XIII) : 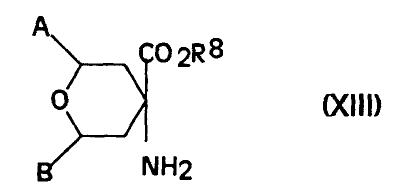 dans laquelle :
A et B ont les significations indiquées à la revendication 1,
et
R⁸ représente un radical alcoyle.

7. Composés de la formule (XV) : 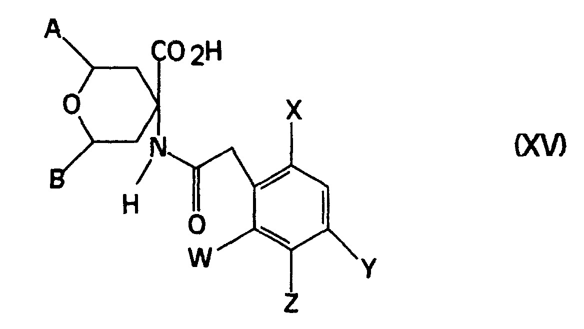 dans laquelle :
A, B, W, X, Y et Z ont les significations indiquées à la revendication 1.

8. Composés de la formule (XVI) : 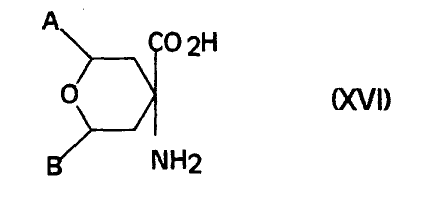 dans laquelle :
A et B ont les significations indiquées à la revendication 1.

9. Composés de la formule (XXII) : 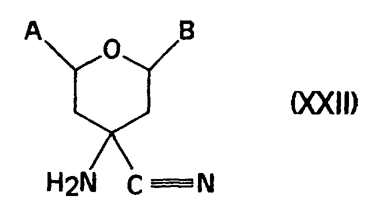 dans laquelle :
A et B ont les significations indiquées à la revendication 1.

10. Composés de la formule (XXIII) : 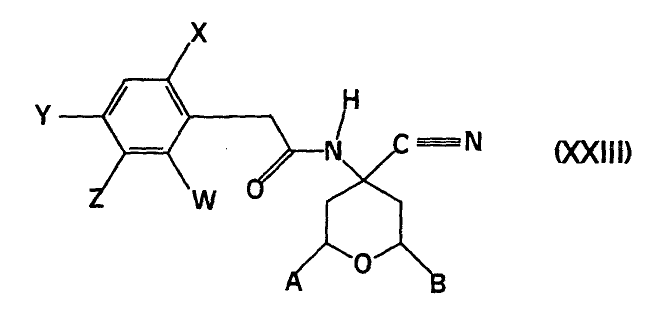 dans laquelle :
A, B, X, Y, Z et W ont les significations indiquées à la revendication 1.

11. Agent de lutte contre les parasites ou agent herbicide, **caractérisé par** une teneur en un composé de la formule (I) suivant la revendication 1.

12. Utilisation des composés de la formule (I) suivant la revendication 1, pour lutter contre des parasites et des mauvaises herbes.

13. Procédé pour lutter contre des parasites et des mauvaises herbes, **caractérisé en ce que** l'on fait agir des composés de la formule (I) suivant la revendication 1, sur les parasites ou mauvaises herbes et/ou leur biotope.

14. Procédé de préparation d'agents de lutte contre des parasites et d'agents herbicides, **caractérisé en ce que** l'on mélange des composés de la formule (I) suivant la revendication 1, avec des diluants et/ou agents tensioactifs.
